(19) Europäisches Patentamt
European Patent Office
Office européen des brevets

(11) **EP 4 190 253 A1**

(12) **EUROPEAN PATENT APPLICATION**
published in accordance with Art. 153(4) EPC

(43) Date of publication:
**07.06.2023 Bulletin 2023/23**

(21) Application number: **21851302.6**

(22) Date of filing: **30.07.2021**

(51) International Patent Classification (IPC):
**A61B 17/34** (2006.01)    **A61M 5/42** (2006.01)

(52) Cooperative Patent Classification (CPC):
**A61B 17/34; A61M 5/42**

(86) International application number:
**PCT/JP2021/028462**

(87) International publication number:
**WO 2022/025284 (03.02.2022 Gazette 2022/05)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.07.2020   JP 2020129188**

(71) Applicants:
• **Ochanomizu University**
  **Bunkyo-ku**
  **Tokyo 112-8610 (JP)**
• **Juntendo University**
  **Tokyo 113-8421 (JP)**

(72) Inventors:
• **OHTA, Yuji**
  **Tokyo 112-8610 (JP)**
• **MIMORI, Ayane**
  **Tokyo 112-8610 (JP)**
• **AKIBA, Chihiro**
  **Tokyo 136-0075 (JP)**
• **BANDAI, Hideki**
  **Tokyo 136-0075 (JP)**
• **MIYAJIMA, Masakazu**
  **Tokyo 136-0075 (JP)**

(74) Representative: **Herrero & Asociados, S.L.**
  **Cedaceros, 1**
  **28014 Madrid (ES)**

(54) **GUIDE DEVICE AND MANUFACTURING METHOD THEREFOR**

(57) Provided is a guide device for puncturing with a puncture needle from a puncture point A provided at a vicinity of a first point B on a waist of a patient to a target point D inside a patient's body, the target point D corresponding to a second point C different from the first point B on the waist. The guide device includes a guide part for guiding the puncturing with the puncture needle, and a support base for supporting the guide part and abutting on the waist. A portion of the support base is configured to define a first angle with respect to a straight line connecting the puncture point A and the second point C, and the guide part is provided at a second angle with respect to a line perpendicular to the support base. The first angle is defined by the puncture point A, the second point C and the first point B, and the second angle is defined by the puncture point A, the target point D and the second point C. Accordingly, it is possible to provide a guide device capable of easily and accurately puncturing a target point with a puncture needle regardless of a patient or an operator.

【FIG.5】

FIG.5

**Description**

**TECHNICAL FIELD**

The present invention relates to a guide device and a method for manufacturing the guide device, and more particularly to a guide device for a puncture needle into a lumbar subarachnoid space and a method for manufacturing the guide device.

**BACKGROUND ART**

Idiopathic normal pressure hydrocephalus (iNPH) is a disease unique to an elderly person, of which main symptoms (three signs) are walking disturbance, dementia and urinary incontinence. When patients suffer from the iNPH, their quality of life (QOL: quality of life) is impaired, such as difficulty in walking, serious forgetfulness and difficulty using a toilet. Therefore, proper diagnosis and treatment of the iNPH for the patients suffering from these symptoms are very important for the patients and caregivers such as their family members to obtain better lives.
In the treatment of the iNPH, an operation called "cerebrospinal fluid shunt" is performed to improve a flow of cerebrospinal fluid. This operation is performed by implanting a catheter (tube) in a poorly flowing cerebrospinal fluid passageway and continually eliminating the cerebrospinal fluid being excess in the cerebrospinal space from the catheter. This makes it possible to release pressure on the brain by the cerebrospinal fluid and improve the cerebrospinal fluid circulation and the brain function.
Currently in Japan, "ventriculo-peritoneal shunt surgery" in which a small hole is made in the skull and the catheter is inserted from a cerebral ventricle to a peritoneal space, and "lumbar subarachnoid-peritoneal shunt surgery" in which the catheter is inserted from the lumbar subarachnoid space to the peritoneal space are frequently performed. Especially, the lumbar subarachnoid-peritoneal shunt surgery is frequently performed for the elderly person from the viewpoint that a burden on the patient is small. The insertion of the catheter in this surgical method is performed by a method (median approach) of inserting a puncture needle at a right angle from a median line of a patient's body.
However, in this median approach, it is difficult for the puncture needle to reach the target point due to an influence of a bone structure. Therefore, it is also difficult to insert the catheter through the puncture needle. In addition, the insertion of the catheter into the lumbar subarachnoid space during the lumbar subarachnoid-peritoneal shunt surgery is largely dependent on a skill of an operator. Especially in the elderly person, a space between spinous processes, which is an insertion path of the catheter, is often narrowed by a deformation of a spinal canal structure with aging. In addition, it is difficult for the patient to take a posture that expands the space between the spinous processes. Therefore, since the puncture needle is difficult to reach the target point and the catheter is difficult to be inserted through the puncture needle, there is a problem that the lumbar subarachnoid-peritoneal shunt surgery for the elderly person is highly difficult.
In addition, as a method other than the median approach, there is a method (paramedian approach) of inserting a puncture needle from a predetermined skin point deviated from the median line. This paramedian approach is a method in which the target point can be punctured with the puncture needle without being affected by the spinous processes. However, even when the paramedian approach is used, it is not easy to accurately puncture the target point with the puncture needle.
Therefore, there is a demand for a technique capable of easily and accurately inserting the catheter into the lumbar subarachnoid space regardless of a patient and an operator. Particularly in Japan where the aging of the population has rapidly progressed and the iNPH medical treatment is advanced, such a technique attracts attention.

**RELATED ART DOCUMENT**

**PATENT DOCUMENT**

Patent Document 1 is JP H7-250902 A
Patent Document 2 is JP 2019-213662 A

**SUMMARY OF THE INVENTION**

**PROBLEMS TO BE SOLVED BY THE INVENTION**

The inventors of the present invention have developed a guide device capable of reliably puncturing a target point with a puncture needle without being affected by spinous processes when using a paramedian approach for insertion of a catheter into a lumbar subarachnoid space by lumbar subarachnoid-peritoneal shunt surgery.

That is, an object of the present invention is to provide a guide device capable of easily and accurately puncturing a target point with a puncture needle regardless of a patient and an operator. Another object of the present invention is to provide a method of manufacturing the guide device.

## MEANS OF SOLVING THE PROBLEMS

The above objects can be achieved by the present invention described in items (1) to (12) below.

(1) A guide device for puncturing with a puncture needle from a puncture point A provided at a vicinity of a first point B on a waist of a patient to a target point D inside a patient's body, the target point D corresponding to a second point C different from the first point B on the waist, the guide device comprising:

a guide part for guiding the puncturing with the puncture needle; and
a support base for supporting the guide part and abutting on the waist,
wherein a portion of the support base is configured to define a first angle with respect to a straight line connecting the puncture point A and the second point C, and
wherein the guide part is provided at a second angle with respect to a line perpendicular to the support base.

(2) The guide device according to the above item (1), wherein the first angle is defined by the puncture point A, the second point C and the first point B, and
wherein the second angle is defined by the puncture point A, the target point D and the second point C.

(3) The guide device according to the above item (1) or (2), wherein the first angle is 20° to 40°.

(4) The guide device according to any one of the above items (1) to (3), wherein the second angle is 10° to 50°.

(5) The guide device according to any one of the above items (1) to (4), wherein the first angle and the second angle are determined by obtaining at least a CT image of the patient including the puncture point A, a CT image of the patient including the target point D, and CT images of the patient between the CT image of the patient including the puncture point A and the CT image of the patient including the target point D.

(6) The guide device according to any one of the above items (1) to (5), wherein the support base is located along a median line of the waist and has a reference portion having a predetermined length.

(7) The guide device according to the above item (6), wherein the support base further includes:

a notch through which the puncture needle passes; and
a puncture guide portion which is provided between the reference portion and the notch and is configured to guide the puncture point A through which the puncture needle is inserted into the patient's body.

(8) The guide device according to the above item (7), wherein the puncture guide portion is orthogonal to the reference portion and has a predetermined length.

(9) The guide device according to any one of the above items (1) to (8), further comprising a first angle changing device for changing the first angle and/or a second angle changing device for changing the second angle.

(10) The guide device according to any one of the above items (1) to (9), wherein the guide part has a plurality of guide parts, and
wherein the second angle includes a plurality of different second angles, and the plurality of guide parts are provided to be supported by the support base with the different second angles respectively.

(11) A method of manufacturing a guide device for puncturing with a puncture needle into a waist of a patient, the method comprising:

irradiating the waist with X-rays to obtain CT images of the waist;
setting a first point B on a median line of the patient, a puncture point A positioned at a vicinity of the first point B, a second point C different from the first point B on the median line and a target point D in the waist corresponding to the second point C to the CT images;

calculating a first angle ∠BCA(θ1) and a second angle ∠CDA(θ2);

preparing a support base designed to define the first angle and a guide part designed to define the second angle; and

connecting the guide part to the support base so as to set an angle with respect to a line perpendicular to the support base to the second angle.

(12) The method of manufacturing the guide device according to the above item (11), further comprising changing the angle of the guide part connected to the support base in accordance with the second angle of the patient.

## EFFECTS OF THE INVENTION

According to the present invention, by setting two angles that determine a spatial mutual positional relationship between a puncture needle and a point of a patient's body, it is possible to easily puncture the target point with the puncture needle without interference of a bone structure. This also facilitates insertion of a catheter. Further, since puncturing angles become gentle, the catheter can be gently inserted into the patient's body, and a risk of damage to the catheter can be suppressed. As described above, according to the present invention, it is possible to provide a guide device capable of easily and accurately puncturing the target point with the puncture needle regardless of a patient and an operator. Further, according to the present invention, it is possible to provide a method of manufacturing the guide device.

## BRIEF DESCRIPTION OF THE DRAWINGS

FIG. 1 is a schematic side view of puncturing a lumbar subarachnoid space.

FIG. 2 is a cross-sectional view schematically showing a CT image of a waist.

FIG. 3 is a schematic view for explaining angles (θ1, θ2) of the puncturing the lumbar subarachnoid space.

FIG. 4 is a perspective view showing a guide device according to a first embodiment of the present invention.

FIG. 5 is a perspective view showing various angles of the guide device according to the first embodiment of the present invention.

FIG. 6 is a flowchart of a method for puncturing with a puncture needle using the guide device according to the first embodiment of the present invention.

FIG. 7 is a perspective view schematically showing a use state of the guide device according to the first embodiment of the present invention.

FIG. 8 is a perspective view schematically showing the guide device according to the first embodiment of the present invention in use.

FIG. 9 is a flowchart showing a method of manufacturing the guide device according to the first embodiment of the present invention.

FIG. 10 includes FIG. 10(a) which is a target slice image and FIG.10(b) which is a cross-sectional view showing a boundary line between a spinal cord and a subarachnoid space in a spinal canal on the target slice image.

FIG. 11 is a perspective view schematically illustrating a puncture state in which a puncture needle passes between a puncture point and a target point in a plurality of slice images.

FIG. 12 is a diagram illustrating a bone region in a CT image.

FIG. 13 includes FIG. 13(a) which is a target slice image and FIG. 13(b) which is a cross-sectional view (partially enlarged view of FIG. 13(a)) schematically showing a boundary line between a spinal cord and a subarachnoid space in a spinal canal on the target slice image.

FIG. 14 is a perspective view schematically showing a reachable range of a tip of the puncture needle.

FIG. 15 is a schematic diagram for explaining a method of calculating a first angle and a second angle.

FIG. 16 is a schematic diagram for explaining the method of calculating the first angle and the second angle.

FIG. 17 is a flowchart illustrating a method of automatically determining a puncture point A and a target point D.

FIG. 18 is a schematic diagram illustrating another configuration example for explaining the method of calculating the first angle and the second angle.

FIG. 19 is a perspective view schematically showing a guide device according to a second embodiment of the present invention.

FIG. 20 is a perspective view schematically showing a guide device according to a third embodiment of the present invention.

FIG. 21 is a perspective view schematically showing a guide device according to a fifth embodiment of the present invention.

FIG. 22 is a side view schematically showing a modified example of the guide device according to the fifth embodiment of the present invention.

FIG. 23 is a perspective view schematically showing a guide device according to a sixth embodiment of the present invention.

FIG. 24 is a top view schematically showing a modified example of the guide device according to the sixth embodiment of the present invention.

FIG. 25 is a perspective view schematically showing a guide device according to a seventh embodiment of the present invention.

FIG. 26 is a perspective view schematically showing a guide device according to an eighth embodiment of the present invention.

## MODE FOR CARRYING OUT THE INVENTION

Hereinafter, a guide device of the present invention will be described in detail with reference to preferred embodiments shown in the accompanying drawings. For convenience of explanation, the upper side in each of the drawings is described as "upper", the lower side in the drawing is described as "lower", the left side in the drawing is described as "left", the right side in the drawing is described as "right", the front side of the drawing is described as "front", and the rear side of the paper is described as "rear".

### 1. Lumbar subarachnoid-peritoneal shunt surgery

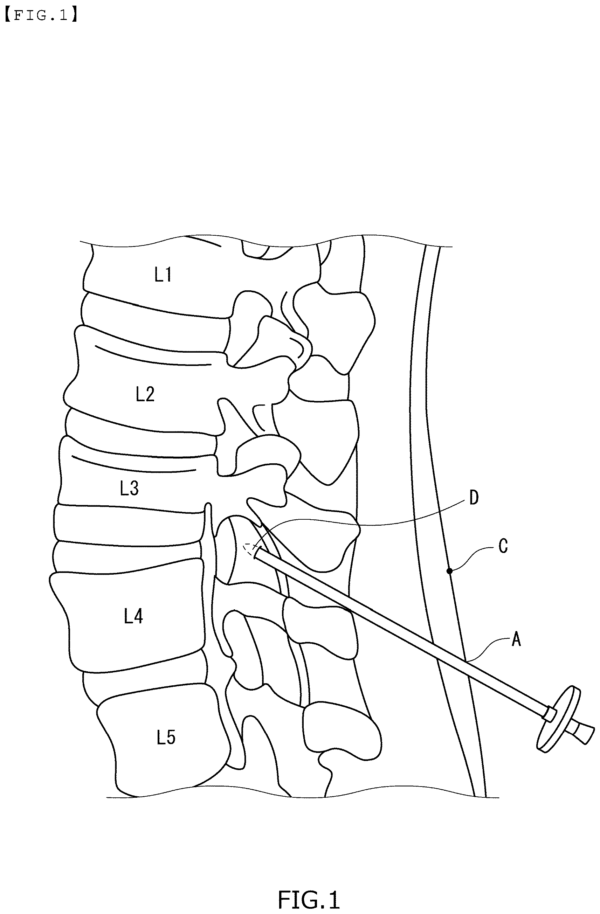

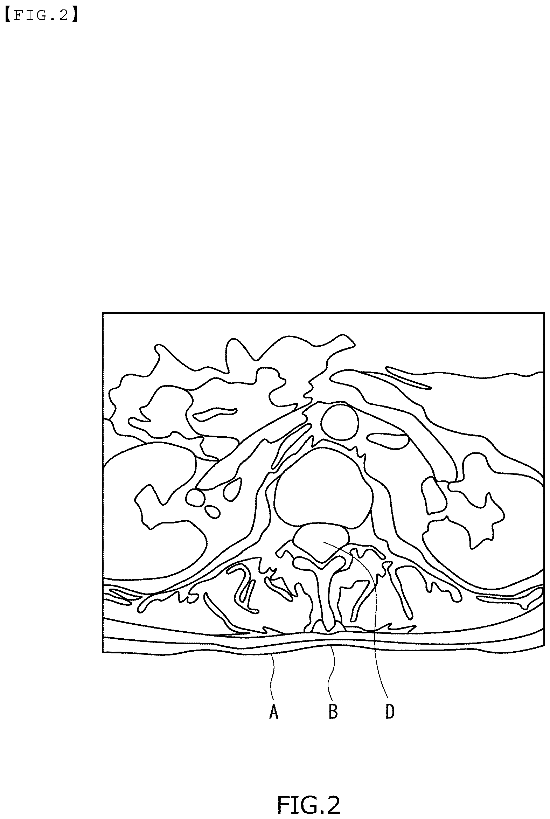

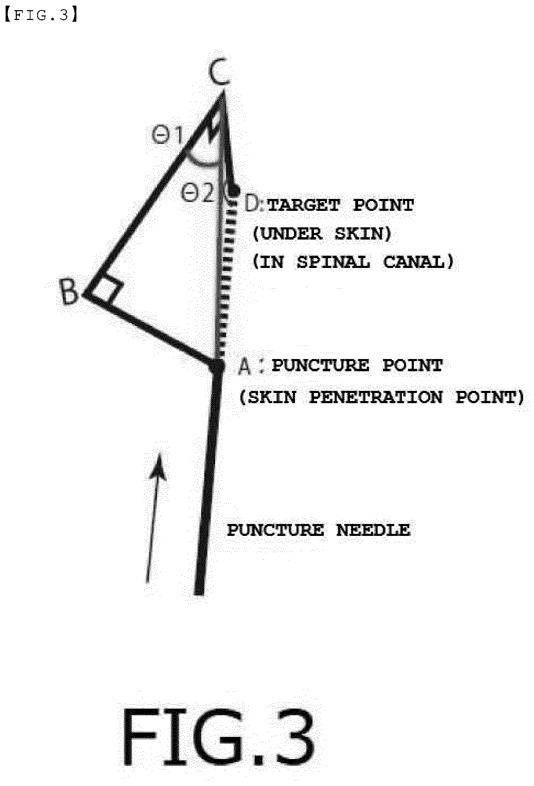

First, a lumbar subarachnoid-peritoneal shunt surgery is described. FIG. 1 is a schematic side view of puncturing a lumbar subarachnoid space. FIG. 2 is a cross-sectional view schematically showing a CT image of a waist. FIG. 3 is a schematic view for explaining angles of the puncturing the lumbar subarachnoid space.
The lumbar subarachnoid-peritoneal shunt surgery is a treatment that allows continuous flow of cerebrospinal fluid from a lumbar subarachnoid space into a peritoneal space. Specifically, the lumbar subarachnoid-peritoneal shunt surgery connects the lumbar subarachnoid space and the peritoneal space with a silicone tube (catheter). The tube passes under the skin and extends from the lumbar subarachnoid space to the peritoneal space. The cerebrospinal fluid in the lumbar subarachnoid space is then guided into the peritoneal space to be absorbed in the peritoneal space. In this surgical method, as shown in FIG. 1, the puncturing with a puncture needle is performed from a skin point corresponding to a vicinity of a position between a spinous process of a fourth lumbar vertebra (L4) and a spinous process of a fifth lumbar vertebra (L5). This allows the puncture needle to reach the lumbar subarachnoid space. In this state, an inner cylinder of the puncture needle is removed, and the tube for removing the cerebrospinal fluid is inserted from an outer cylinder of the puncture needle. The tube is then passed through the peritoneal space by a surgical procedure.

The lumbar subarachnoid-peritoneal shunt surgery is generally performed by puncturing with a needle at a skin point (puncture point) A that is a predetermined distance (e.g., 15 mm) away in a direction perpendicular to a median line from a skin point (first point) B on the median line that corresponds to the position between the spinous process of the fourth lumbar vertebra and the spinous process of the fifth lumbar vertebra. That is, the puncturing with the needle is performed at the skin point A that is away from the median line and between the spinous process of the fourth lumbar vertebra and the spinous process of the fifth lumbar vertebra. The puncture needle reaches the lumbar subarachnoid space (in the spinal canal) corresponding to a position between the third lumbar vertebra and the fourth lumbar vertebra. The cerebrospinal fluid is collected and the tube is inserted through the puncture needle.

As a result of intensive studies of this lumbar subarachnoid-peritoneal shunt surgery, the inventors have completed the present invention with the aim of developing a device capable of easily puncturing a target point with a needle regardless of a patient and an operator. Specifically, in the lumbar subarachnoid-peritoneal shunt surgery, CT images of the lumbar spinal canal as shown in FIGs. 1 and 2 are acquired prior to the puncturing with the puncture needle. Then, the inventors have considered that a positional relationship between the puncture point A, the skin point B on the median line corresponding to the position between the spinous process of the fourth lumbar vertebra and the spinous process of the fifth lumbar vertebra, the skin point (second point) C on the median line corresponding to the position between the spinous process of the third lumbar vertebra and the spinous process of the fourth lumbar vertebra and the target point D is important.

FIG. 3 schematically represents these points and puncturing angles. A line BC refers to the median line of a patient's body. The puncture point A is a point which is spaced from the skin point B by 15 mm and on a line perpendicular to the line BC. CT images of the lumbar spinal canal of each of many patients have been acquired and these points have been set on the CT images. As a result, the inventors have confirmed a fact that each of the $\angle ACB$ (first angle $\theta 1$) and the $\angle CDA$ (second angle $\theta 2$) is approximately 30°. In view of the above, the inventors have developed the device capable of easily puncturing the target point with the needle regardless of the patient and the operator by utilizing this fact.

Further, the inventors have developed a method capable of automatically setting the puncture point A spaced from the median line by a predetermined distance, the target point D, the first angle $\theta 1$ and the second angle $\theta 2$ for each patient without setting them in advance.

<<1 st embodiment >>

2. Guide device

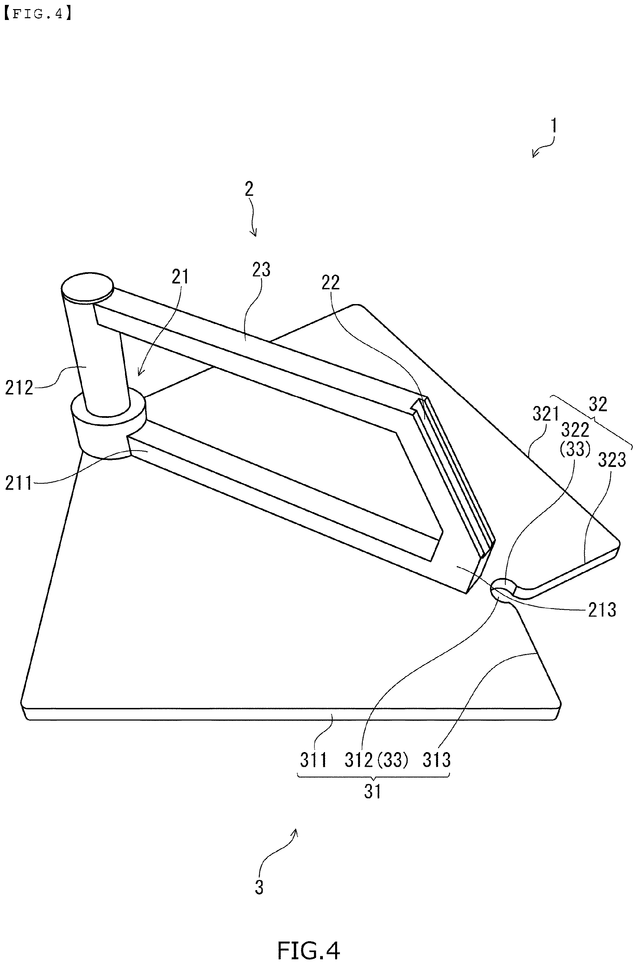

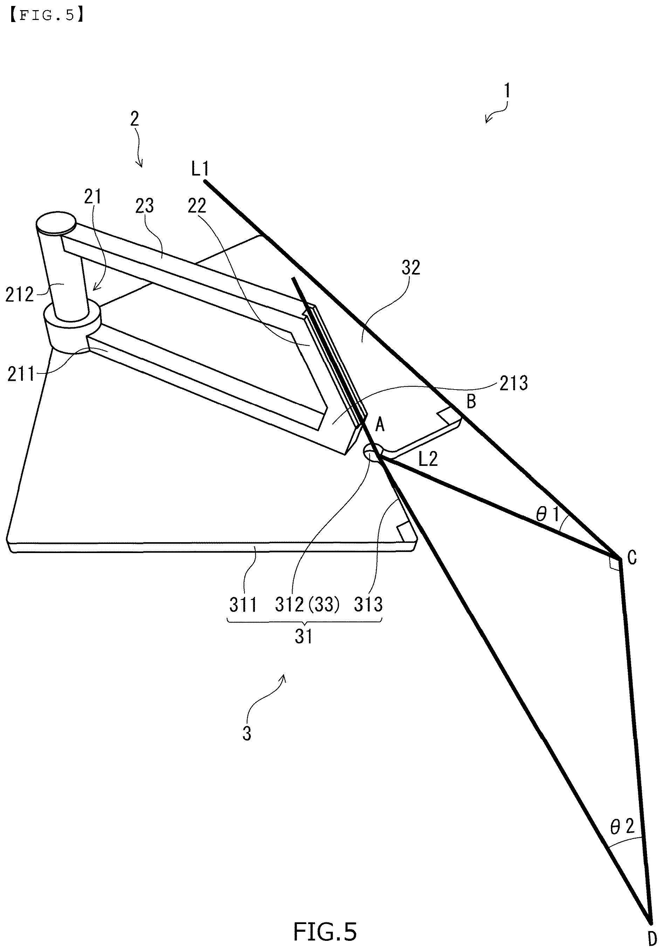

Next, the guide device of the present invention will be described in detail. FIG. 4 is a perspective view showing a guide device according to a first embodiment of the present invention. FIG. 5 is a perspective view showing various angles of the guide device according to the first embodiment of the present invention.

As shown in FIG. 4, a guide device 1 of the present invention is a device that guides the puncturing into the lumbar subarachnoid space. The guide device 1 includes a guide part 2 and a support base 3. Hereinafter, a configuration of each part of the guide device 1 will be described.

<Guide part>

The guide part 2 has a function of guiding the puncturing with the puncture needle. The shape of the guide part 2 is not particularly limited, and examples thereof include a trapezoidal shape, a cuboid shape, a triangular columnar shape, an elliptical columnar shape, and a cylindrical columnar shape. The guide part 2 includes a main body 21, a guide rail 22, and a grip portion 23.

(Main body)

The main body 21 has a function of supporting the guide rail 22 and the grip portion 23 on the support base 3 described later. The main body 21 is formed into a substantially L-shaped structure, but is not limited thereto. For example, the main body 21 may be a truncated cone or a structure with two trapezoidal faces. The main body 21 is formed of a transparent member, but is not limited thereto. In the present embodiment, the main body 21 includes a reinforcing portion 211, a first support portion 212 and a second support portion 213 smaller than the first support portion 212. The reinforcing portion 211 reinforces the connection between the guide part 2 and the support base 3. The reinforcing portion 211 is formed into an elongated shape, but is not particularly limited.

The first support portion 212 stands from a proximal end of the reinforcing portion 211. The second support portion 213 stands from a distal end of the reinforcing portion 211. The height of the first support portion 212 may be different from the height of the second support portion 213. In order to angle the guide rail 22 with respect to the support base 3, the length of the second support portion 213 is preferably 1/2 to 1/4 of the length of the first support portion 212. Note that the main body 21 may not have the second support portion 213. In this case, the guide rail 22 is directly connected to the support base 3.

(Guide rail)

The guide rail 22 has a function of advancing the puncture needle at a predetermined angle with respect to the support base 3. The guide rail 22 is provided to connect the second support portion 213 of the main body 21 and the grip portion 23. As shown in FIG. 5, the guide rail 22 is provided at a predetermined angle with respect to the support base 3. The predetermined angle is preferably 40° to 80°, more preferably 45° to 70° and most preferably 60°. In other words, the guide rail 22 is provided at a second angle with respect to a line perpendicular to the support base 3. As described later, the second angle is preferably 10° to 50°, more preferably 20° to 45° and most preferably 30°. With such an angle, the puncture needle can reliably reach the target point D.

Further, since the puncturing the lumbar subarachnoid space can be performed with the puncture needle at the predetermined angle, subsequent catheter insertion is facilitated. In particular, even in an elderly person whose spinal canal structure has deformed due to aging, the puncturing the lumbar subarachnoid space can be accurately and reliably performed with the puncture needle, and the subsequent catheter insertion can be facilitated.

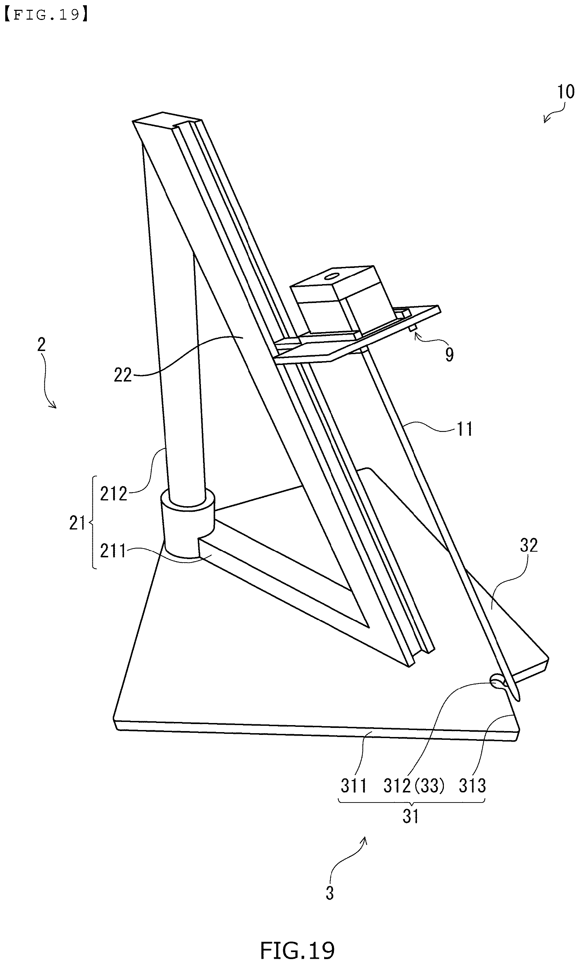

The shape of the guide rail 22 is not particularly limited, and may be planar, semi-cylindrical, cylindrical or the like. In the present embodiment, the guide rail 22 is formed to have a groove. In this case, since an upper side of the guide rail 22 is open, the guide device 1 can be easily removed from an affected area in a state where the puncture needle is inserted into the patient's body through the guide rail 22. The guide rail 22 may have a fixing member 9 (FIG. 19) for fixing the puncture needle, and the fixing member 9 is provided in the groove. The fixing member 9 is configured to be movable along the guide rail 22. Therefore, when the puncture needle is fixed to the fixing member 9, the puncture needle can accurately and reliably reach the puncture point A.

The length of the guide rail 22 is not particularly limited as long as the puncture needle can be guided. However, the guide rail 22 preferably has a length of about 2 cm to 10 cm and more preferably has a length of about 3 cm to 6 cm. As a result, the puncture needle can be reliably guided to the puncture point A. Further, even when the fixing member 9 as described above is attached thereto, the puncture needle can be reliably guided into the patient's body.

A distal end of the guide rail 22 is connected to the second support portion 213. Therefore, the distal end of the guide rail 22 is spaced from the support base 3 by a predetermined distance vertically upward. As a result, it is possible to prevent the guide rail 22 from being damaged by the puncture needle and a constituent material of the guide rail 22 from accidentally entering the patient's body. The predetermined distance corresponds to the length of the second support portion 213 and is preferably about 0.5 cm to 2 cm.

Further, the distal end of the guide rail 22 is spaced by a predetermined distance toward the first support portion 212 along a lengthways direction of the reinforcing portion 211. Thus, even if the member for fixing the puncture needle is attached to the guide rail 22, the puncture needle can be reliably passed through a notch 312 (puncture point A). The predetermined distance is preferably about 0.1 cm to 2 cm from the notch 312.

(Grip portion)

The grip portion 23 has a function of allowing an operator to hold the guide device 1. The grip portion 23 is configured to connect a proximal end of the guide rail 22 and an upper end of the first support portion 212. The grip portion 23 is provided parallel to the reinforcing portion 211 and perpendicular to the first support portion 212. As a result, the main body 21 is formed into a trapezoidal shape as a whole. The thickness and length of the grip portion 23 are not particularly limited as long as the operator can grip the grip portion 23. The grip portion 23 may have a concave portion formed along a finger of the operator so that the operator can securely grip the grip portion 23.

In addition, the grip portion 23 may have a cushioning portion that absorbs a gripping force of the operator. The cushioning portion is formed into an arcuate shape so as to connect a distal end and a proximal end of the grip portion 23 at a lower portion of the grip portion 23. Therefore, when the operator grips the grip portion 23, the operator can obtain a comfortable grip feeling by the cushioning portion. In addition, the cushioning portion may have a concave portion formed along the finger of the operator so that the operator can securely grip the cushioning portion.

<Support base>

The support base 3 has a function of supporting the guide part 2. The support base 3 is formed into a hexagonal plate shape, but is not limited thereto. For example, the support base 3 may have a circular plate shape, a triangular plate shape or a cuboid shape. The support base 3 is provided below the guide part 2. With such a configuration, the operator can grip the grip portion 23 of the guide part 2 and reliably bring the support base 3 into contact with the patient's body. The support base 3 is composed of a first blade portion 31 and a second blade portion 32.

(First blade portion)

The first blade portion 31 is provided so as to protrude from the guide part 2 in one direction perpendicular to the lengthways direction of the reinforcing portion 211 of the guide part 2. The first blade portion 31 is formed into a rectangular plate shape. This ensures that the first blade portion 31 is placed on the patient's body. The first blade portion 31 has a reference portion 311, a notch 312 and a puncture guide portion 313.

The reference portion 311 has a function of placing the guide device 1 along the median line of the patient's body. The reference portion 311 is formed into an elongated shape and constitutes a long side of the first blade portion 31. The length of the reference portion 311 is not particularly limited as long as the reference portion 311 can be placed along the median line. For example, the length of the reference portion 311 is preferably 1 cm to 20 cm, more preferably 3 cm to 15 cm and even more preferably 5 cm to 15 cm. With such a length, the reference portion 311 can be securely placed along the median line.

The reference portion 311 has a distal end and a proximal end thereof. The distal end is provided on the first blade portion 31 so as to protrude in the front side direction (toward the notch 312) from the guide part 2 in the entire guide device 1. The distal end is positioned at the skin point B on the median line corresponding to the location between the spinous process of the fourth lumbar vertebra and the spinous process of the fifth lumbar vertebra. Therefore, the distal end of the reference portion 311 may be formed into a special shape (for example, a star shape, a circular shape or an arrow shape) so as to clearly indicate the skin point B.

The proximal end (rear end) of the reference portion 311 is provided on the first blade portion 31 so as to protrude in a direction perpendicular to the guide part 2 in the entire guide device 1. The rear end of the reference portion 311 is placed on the median line. The shape and configuration of the rear end of the reference portion 311 are not particularly limited, and may be any shape and configuration.

The notch 312 has a function of passing the puncture needle therethrough. The notch 312 is provided on an extension line of the guide rail 22 and directly below the guide rail 22. As a result, the puncture needle that has advanced along the guide rail 22 can reliably pass through the notch 312. The notch 312 is formed into a substantially 1/4 circle shape in a plan view of the first blade portion 31. In other words, the notch 312 is formed into a concave shape. Therefore, after puncturing the patient's body with the puncture needle through the notch 312, the guide device 1 can be easily removed. Note that the notch 312 may be a through hole formed into the first blade portion 31, and the size of the notch 312 is not particularly limited as long as the puncture needle can pass therethrough.

The puncture guide portion 313 has a function of indicating an actual location of the puncture point A. The puncture guide portion 313 is provided between the reference portion 311 and the notch 312. The puncture guide portion 313 is formed into an elongated shape so as to be perpendicular to the reference portion 311 and connected to the notch 312. Therefore, one end of the puncture guide portion 313 constitutes a vertex (distal end) of the first blade portion 31 together with the distal end of the reference portion 311. The other end of the puncture guide portion 313 constitutes another vertex of the first blade portion 31 together with the notch 312.

The guide device 1 is placed on the patient's body so that the distal end of the reference portion 311 is aligned with the skin point B and the reference portion 311 is aligned with the median line. As a result, the puncture guide portion 313 can indicate the puncture point A spaced from the median line by the predetermined distance. Therefore, as long as the one end and the other end of the puncture guide portion 313 are configured as described above, a portion between the one end and the other end thereof may be configured in any manner. For example, the portion may be curved or protruding. The length of the puncture guide portion 313 is preferably about 3 mm to 20 mm, more preferably about 5 mm to 18 mm and most preferably 15 mm. As a result, the puncture point A suitable for the puncturing can be reliably indicated.

As shown in FIGs. 4 and 5, a reference portion 321 is configured such that an extension line L1 of the reference portion 321 intersects an imaginary line L2 passing through a notch 322 (an extension line of the reference portion 311 is similar thereto). That is, as shown in FIG. 5, the reference portion 321 is configured such that the line BC intersects the line AC at a predetermined angle (first angle $\angle$BCA) $\theta$1. The predetermined angle $\theta$1 of the reference portion 321 is preferably 20° to 40° and more preferably 30°. This allows the puncture needle to reliably reach the target point D. The imaginary line L2 is defined along a lengthways direction of the guide part 2 or a surface direction of the support base 3.

Further, the extension line of the guide rail 22 formed into the elongated shape intersects the line perpendicular to the support base 3. That is, as shown in FIG. 5, the extension line is configured to intersect a line CD at a predetermined angle (second angle $\angle$CDA) $\theta$2. The predetermined angle $\theta$2 is preferably 10° to 50°, more preferably 20° to 45° and most preferably 30°. This allows the puncture needle to reliably reach the target point D. The line CD is perpendicular to the line AC.

(Second blade portion)

The second blade portion 32 is provided so as to protrude from the guide part 2 in a direction perpendicular to the lengthways direction of the guide part 2 and in a direction opposite to the first blade portion 31. Since the configuration of the second blade portion 32 is the same as the configuration of the first blade portion 31, a detailed description thereof will be omitted.

The second blade portion 32 is provided symmetrically to the first blade portion 31 about the reinforcing portion 211 of the guide part 2 as a central axis. That is, the support base 3 is formed line-symmetrically about the guide part 2 as the center axis. Thus, since the guide device 1 has the two reference portions which are the reference portion 311 of the first blade portion 31 and the reference portion 321 of the second blade portion 32, the guide device 1 can be used in both a case of puncturing from a left side of a back of a patient with respect to the median line and a case of puncturing from a right side of the back of the patient with respect to the median line. Therefore, the guide device 1 can be used for any type of patient.

The notch 322 of the second blade portion 32 is formed into a substantially 1/4 circle shape in a plan view of the second blade portion 32, similarly to the notch 312 of the first blade portion 31. Therefore, when viewed in the entire support base 3 including the first blade portion 31 and the second blade portion 32, the notch 312 and the notch 322 (also referred to as the "notch 33 of the support base 3") are formed into a substantially semicircular shape. The notch 33 may be a through hole formed in the support base 3.

The notch 33 of the support base 3 is provided along the lengthways (forward) direction of the guide part 2 between the puncture guide portion 313 of the first blade portion 31 and the puncture guide portion 323 of the second blade portion 32. The notch 33 constitutes a concave portion (bottom point) provided between the distal end (vertex) of the reference portion 311 and the distal end (vertex) of the reference portion 321 in a plan view of the guide device 1. With such a configuration, since the notch 33 is open, the guide device 1 can be easily removed from the patient's body after puncturing the patient's body with the puncture needle. Note that the guide device 1 may not have either the first blade portion 31 or the second blade portion 32.

3. Puncture method using guide device

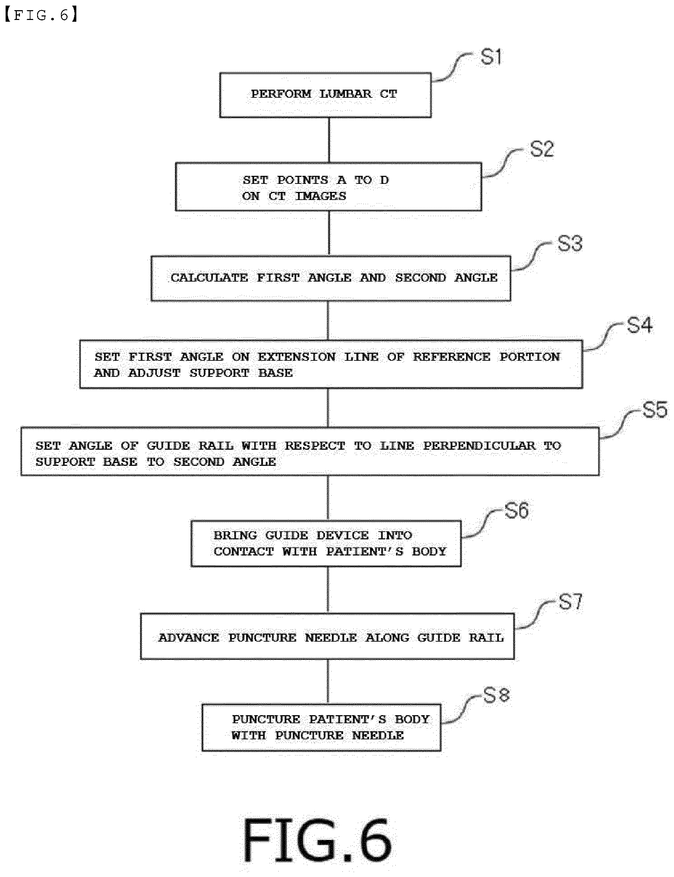

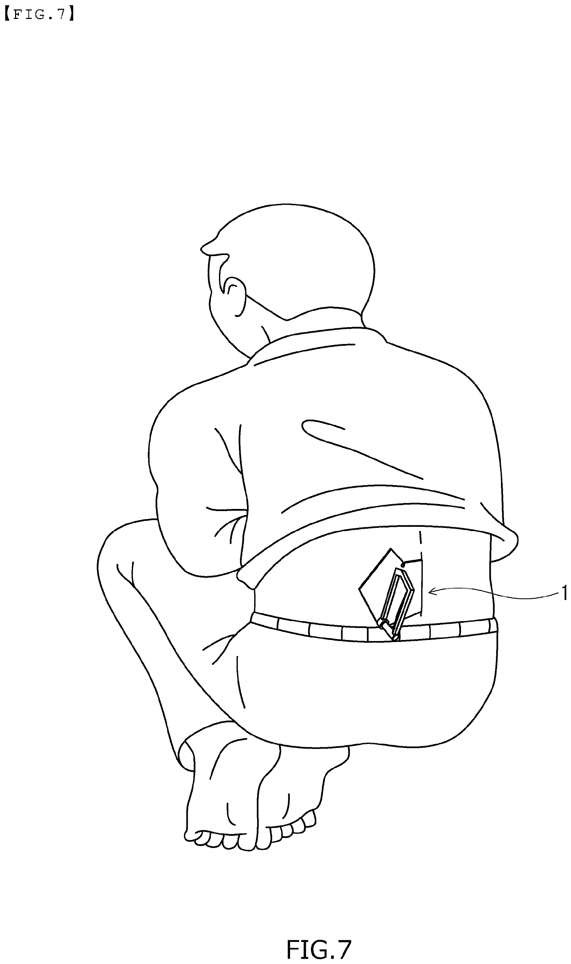

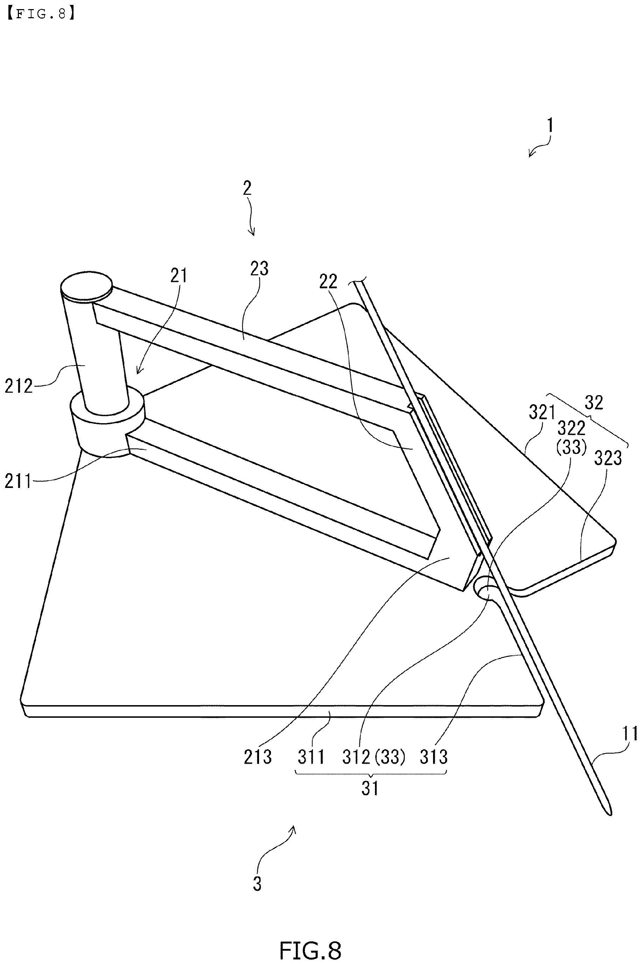

Next, a method for puncturing with a puncture needle using the guide device 1 of the present invention will be described. FIG. 6 is a flowchart of a method for puncturing with a puncture needle using the guide device according to the first embodiment of the present invention. FIG. 7 is a perspective view schematically showing a use state of the guide device according to the first embodiment of the present invention. FIG. 8 is a perspective view schematically showing the guide device according to the first embodiment of the present invention in use.

As shown in FIG. 6, the puncture method of the present invention includes: steps of (S1) irradiating a waist of a patient's body with X-rays; (S2) setting points A to D on CT images; (S3) calculating a first angle and a second angle; (S4) setting the first angle on an extension line of the reference portion and adjusting the support base; (S5) setting an angle of the guide rail with respect to a line perpendicular to the support base to the second angle; (S6) bringing the guide device into contact with the patient's body; (S7) advancing the puncture needle along the guide rail; and (S8) puncturing the patient's body with the puncture needle.

First, in the step S1, a lumbar CT is performed. This is performed using a common CT diagnostic apparatus. Consequently, the CT images as shown in FIG. 1 and FIG. 2 can be obtained. The puncture point A, the skin point B, the skin point C and the target point D are plotted on the obtained CT images (step S2). This may be performed on a computer system or on the CT images. After plotting these points, the first angle $\angle$BCA ($\theta$1) and the second angle $\angle$CDA ($\theta$2) are calculated by connecting the respective points (step S3).

Next, the support base 3 is adjusted so that the angle of intersection between the extension line L1 of the reference portion 321 (311) of the guide device 1 and the imaginary line L2 passing through the notch 33 is set to the obtained first angle $\theta$1 (step S4). For example, the adjustment of the support base 3 can be performed by cutting a portion of the support base 3. Then, the angle of the guide rail 22 with respect to the line perpendicular to the support base 3 of the guide device 1 is set to the obtained second angle $\theta$2 (step S5). Thus, the guide device 1 can be obtained. Thereafter, the support base 3 of the guide device 1 thus obtained is brought into contact with the patient's body (step S6). At this time, as shown in FIG. 7, the intersection point between the reference portion 311 and the puncture guide portion 313 of the guide device 1 of the present invention (the distal end of the reference portion 311) is aligned with the skin point B on the median line corresponding to the position between the spinous process of the fourth lumbar vertebra and the spinous process of the fifth lumbar vertebra. Then, the guide device 1 is located on the patient's body so that the reference portion 311 is located along the median line of a back surface of a patient. Accordingly, the puncture point A is indicated by the puncture guide portion 313, and the puncture point A is positioned at the position of the notch 312 along the guide rail 22.

Thereafter, as shown in FIG. 8, a puncture needle 11 provided in the guide rail 22 is advanced along the guide rail 22 (step S7). This ensures that the puncture needle 11 reaches the puncture point A through the notch 33. Then, the puncture needle 11 is inserted into the patient's body from the puncture point A (step S8). Since the shapes of the guide rail 22 and the notch 33 are open, the guide device 1 can be easily removed from the patient's body when the puncture needle 11 is inserted to some extent (for example, 5 cm). As a result, it is possible to prevent a subsequent operation from being obstructed by the guide device 1. Thereafter, the puncture needle 11 is further advanced manually (for example, 1 cm to 2 cm), and the tip of the puncture needle 11 reaches the target point D as shown in FIG. 1. At this time, the puncture needle 11 has been inserted into the patient's body about 6 cm to 7 cm.

The puncturing is performed by the above-described method. The first angle $\theta 1$ and the second angle $\theta 2$ generally tend to be common in a plurality of patients. Therefore, once the first angle $\theta 1$ and the second angle $\theta 2$ are set in the guide device 1, the steps (S1) to (S5) can be omitted. That is, if the guide device 1 is used, a puncturing procedure can be immediately performed on a patient without performing a CT image diagnosis in advance. Therefore, it is possible to greatly reduce the time required for the operation. As described above, the puncture method of the present invention can be easily performed regardless of the patient and the operator, allowing the puncture needle 11 to reach the target point D quickly and reliably.

Further, according to the puncture method of the present invention, the target point D in the subarachnoid space between the third lumbar vertebra and the fourth lumbar vertebra is punctured with the puncture needle from the puncture point A at the vicinity of the skin point B corresponding between the spinous process of the fourth lumbar vertebra and the spinous process of the fifth lumbar vertebra. That is, according to the puncture method of the present invention, the puncture needle 11 is inserted into the target point D above one intervertebral from the puncture point A. Therefore, multiple puncturing due to incorrect puncturing can be avoided, and complications (e.g., bleeding, prolonged operation time, postoperative rash and the like) can be avoided.

4. Method of manufacturing guide device

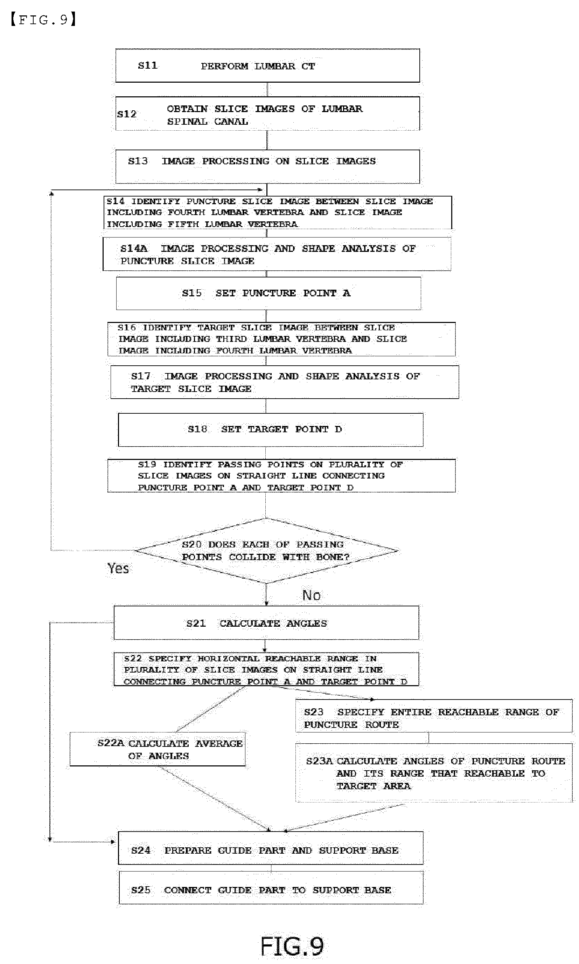

Next, a method of manufacturing the guide device of the present invention will be described. FIG. 9 is a flowchart showing a method of manufacturing the guide device according to the first embodiment of the present invention. As shown in FIG. 9, the method includes a step (S11) of irradiating a waist with X-rays, steps (S12 to S20) of setting the puncture point A, the skin point (first point) B, the skin point (second point) C and the target point D in the CT images, a step (S21) of calculating the first angle $\theta 1$ and the second angle $\theta 2$, a step (S24) of preparing the guide part 2 and the support base 3 and a step (S25) of connecting the guide part 2 to the support base 3. In addition, the method includes a step (S22) of specifying a horizontal reachable range of the puncture needle, a step (S23) of specifying an entire reachable range of the puncture needle and a step (S23A) of calculating angles when passing through the puncture point and a center of the reachable range or an approximate position thereof as angles for manufacturing the guide device. The method including the steps S11 to S23 is also referred to as a method of specifying the reachable range of the puncture needle in the patient's body.

The method of manufacturing the guide device 1 and the method of specifying the reachable range of the puncture needle according to the present invention can be performed manually or automatically using a processing device such as a computer device. Hereinafter, a case where these methods are automatically performed will be described as an example.

First, the processing device used in these methods is connected to a CT diagnostic apparatus and a printer by wire or wirelessly. The processing device includes a control unit that executes each process of the methods, a storage unit that stores predetermined information and a display unit that displays the predetermined information. The predetermined information includes personal information of patients, a plurality of CT images (slice images) obtained by the CT diagnostic apparatus, distance information from the median line to the puncture point A and information related to the angles (first angle $\theta 1$ and second angle $\theta 2$), the first point B, the second point C, the target point D and the like. The operator can perform image processing, angle calculation and the like while visually recognizing the predetermined information displayed on the display unit.

The CT diagnostic apparatus is not particularly limited. The printer is not particularly limited, but may be a three-dimensional printer. By using the three-dimensional printer with a predetermined resin material, the disposable guide device 1 can be quickly manufactured. In addition, since the first angle $\theta 1$ and the second angle $\theta 2$ can be easily adjusted, it is possible to manufacture the guide device 1 suitable for various body shapes of the patients. Note that the printer is not essential.

Next, each step of the method of manufacturing the guide device will be described in order. The step S11 is a step of performing a lumbar CT using the common CT diagnostic apparatus. Consequently, the control unit can obtain a plurality of slice images (an example being shown in FIG. 2), which are created so as to be sliced in a direction orthogonal to the upper and lower direction (vertical direction) in FIG. 1 (step S12). Here, the control unit can automatically set or preset a distance (interval) between adjacent slice images among the plurality of slice images. If this interval is small, the position of the spinal canal and the positions of the lumbar vertebrae can be accurately determined. Therefore, the interval is preferably about 0.5 mm to 1.8 mm.

The control unit executes the image processing on the plurality of obtained slice images (step S13). Examples of the image processing include, but are not limited to, binarization processing and other general processing. Thereby, the control unit sets a contour line around spinous processes in all the slice images. Then, in all the slice images, the control unit identifies the spinous processes of the third to fifth lumbar vertebrae based on peak information of a degree of curvature, a position of the contour line (position of the body in the front-to-rear direction) and the like. Thus, the control unit identifies a plurality of slice images including the spinous processes of the third to fifth lumbar vertebrae.

Next, the control unit identifies an intermediate slice image (puncture slice image) between the slice image including the fourth lumbar vertebra and the slice image including the fifth lumbar vertebra (step S14). Here, the control unit executes the image processing such as the binarization processing of the puncture slice image and shape analysis thereof (step S14A), and extracts a contour line of the skin. Then, in the puncture slice image, the control unit sets a point which is apart from a median plane (plane including the spinous processes) by a predetermined distance (for example, 15 mm) in a transverse direction (left or right direction) along the contour line of the skin as the puncture point A (step S15). That is, as shown in FIG. 2, in the puncture slice image, the control unit sets the point which is apart from the skin point B on the median line by for example 15 mm in left along the contour line of the skin as the puncture point A. Note that this distance is not limited to 15 mm.

After setting the puncture point A, the control unit identifies an intermediate slice image between the slice image including the third lumbar vertebra and the slice image including the fourth lumbar vertebra (step S16). This intermediate slice image is a target slice image which the puncture needle reaches.

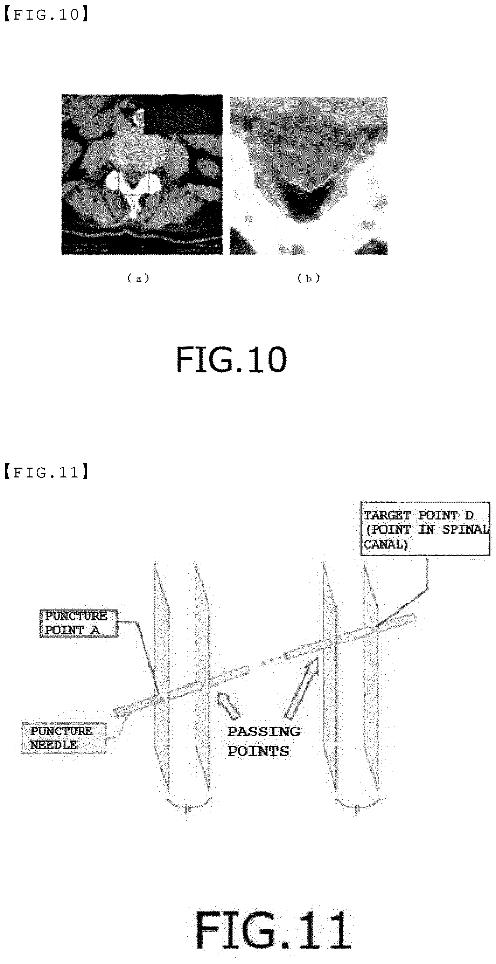

Next, as shown in FIG. 10, the control unit executes the image processing such as the binarization processing of the target slice image and the shape analysis thereof (step S17). This identifies the spinal canal and a back-side boundary line between a spinal cord and the subarachnoid space in the spinal canal. The control unit sets a center point in the spinal canal as the target point D (step S18).

Next, the control unit identifies passing points on the plurality of slice images through which a straight line connecting the puncture point A and the target point D (hereinafter referred to as a "straight line AD") passes (step S19). That is, as shown in FIG. 11, in the plurality of slice images between the puncture slice image including the puncture point A and the target slice image including the target point D, coordinates of each of the passing points through which the straight line AD (puncture needle) passes are calculated. Since each slice image is provided so as to be parallel and equally spaced, the coordinates of the passing point are calculated by proportional calculation. Note that the x and y coordinates in the respective slice images are specified in the step S13.

For example, it is assumed that the x coordinate of the target point D on the target slice image is 238 pix, the x coordinate of the puncture point A on the puncture slice image is 278 pix and the number of the plurality of slice images between the puncture slice image and the target slice image is 49. At this time, the x coordinate of one slice image among the plurality of slice images and the x coordinate of another slice image adjacent thereto are calculated based on the proportional calculation as follows.

· The x coordinate (pix) of the passing point on the one slice image

$$(278\text{-}238) \div (49\text{-}1) + 238 = 238.\ 9375 = 239$$

· The x coordinate of the passing point on the adjacent slice image

$$(278\text{-}238) \div (49\text{-}1) + 239 = 239.\ 875 = 240$$

The x coordinate of the passing point on each of the slice images other than these slice images is also calculated by repeating this calculation. Further, the y coordinate of the passing point on each slice image can be calculated by the similar calculation as the x coordinate.

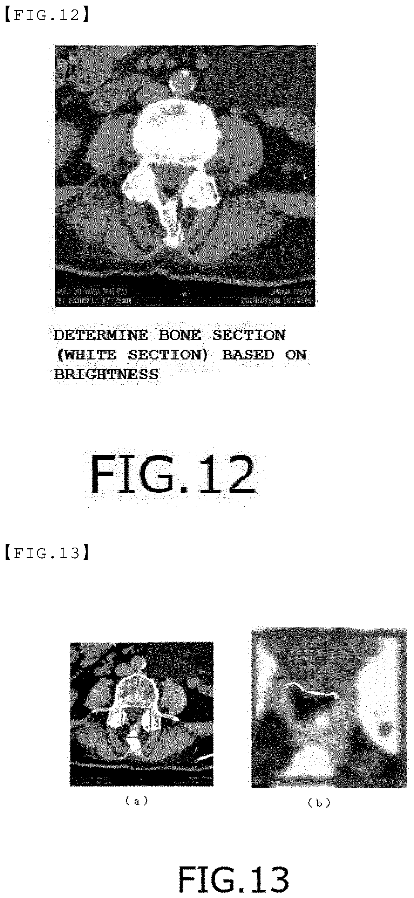

Next, the control unit determines whether or not each of the calculated passing points collides with a bone (step S20). In the determination, as shown in FIG. 12, a bone region in each slice image is determined based on brightness. Specifically, the control unit determines a section where the brightness (0 to 255) on the slice image is equal to or greater than a predetermined value (for example, 230) as the bone by the binarization processing. When the calculated coordinates of the passing point are not included in the bone region, the control unit determines that the passing point does not collide with the bone. This determination is made in consideration of the thickness of the needle. For example, under the condition that an area of 1 mm (thickness of the needle) outward from the boundary of the bone region is defined as an avoidance region, when the coordinates of the passing point are not included in either the bone region or the avoidance region, the control unit determines that the passing point does not collide with the bone. As a result, the puncture point A and the target point D are specified. Then, the process proceeds to the step S21.

On the other hand, when the calculated coordinates of the passing point are included in the bone region, the control unit determines that the passing point collides with the bone. Then, the process returns to the step S14, and the steps S14 to S20 are repeated. That is, the puncture slice image is set to another puncture slice image adjacent thereto, and the puncture point A is set thereon. The target slice image is also set to another target slice image adjacent thereto, and the target point D is set thereon. By repeating this process, the puncture point A and the target point D are specified such that each passing point does not collide with the bone.

In the above description, the x and y coordinates of the passing point at which the straight line AD passes through each slice image have been described. However, in the manufacturing method of the present invention, the x and y coordinates of a passing point at which the puncture needle passes through each slice image may be calculated. In this case, since the puncture needle has a constant thickness, such x and y coordinates are calculated so as to include pixels that are wider than the x and y coordinates of the passing point by the radius of the puncture needle.

In the step S21, the control unit calculates angles. After the step S21, the process proceeds to step S22. The step S22 and the method of calculating the angles will be described later.

Next, the control unit prepares the guide part 2 and the support base 3 based on the angle information obtained in the above-described step S21, and instructs the printer to connect the guide part 2 to the support base 3. Accordingly, the printer can prepare the guide part 2 designed to define the second angle and the support base 3 designed to define the first angle (S24), and connect the guide part 2 to the support base 3 (S25). That is, the printer can manufacture the guide device 1 in which the guide part 2 and the support base 3 are integrated.

When the printer is not used, after the step S21, the guide part 2 and the support base 3 as described above are prepared (S24). The guide part 2 and the support base 3 are made of, for example, stainless steel in advance based on the angle information and the like obtained above. Next, the prepared guide part 2 is connected to the support base 3 by a method such as welding or fitting (S25). Thus, the guide device 1 of the present invention can be manufactured. When the guide device 1 includes an angle changing device 8 described later, the angle of the guide part 2 can be adjusted so as to change the first angle $\theta 1$ or the second angle $\theta 2$ after the step S25.

As described above, the guide device 1 suitable for the various body shapes of the patients can be quickly manufactured. The obtained guide device 1 can be used safely for each patient because the reachable range of the puncture needle in the patient's body is specified based on the slice image information. In addition, according to the method of manufacturing the guide device 1, the puncture point A, the target point D and the straight line AD (path of the puncture needle) can be appropriately determined. Therefore, the guide device 1 of the present invention is particularly useful for puncturing a space such as the lumbar subarachnoid space.

### 5. Method of calculating angles

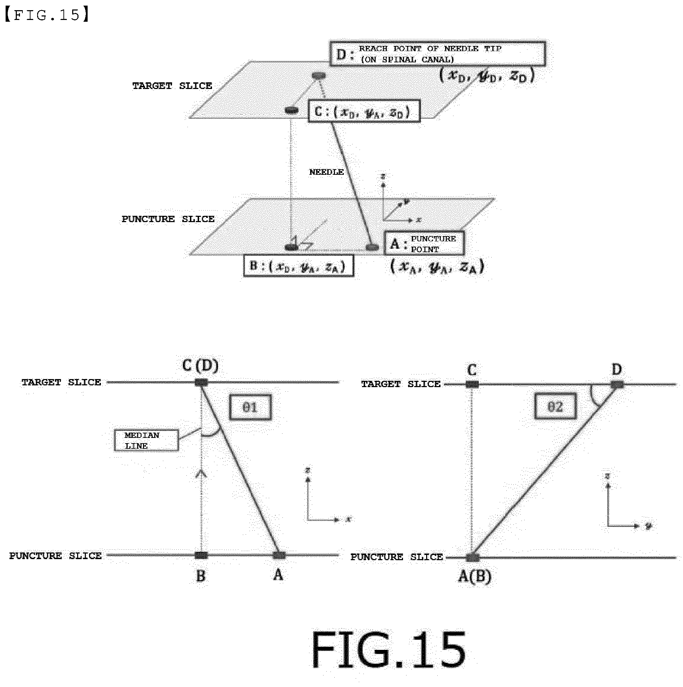

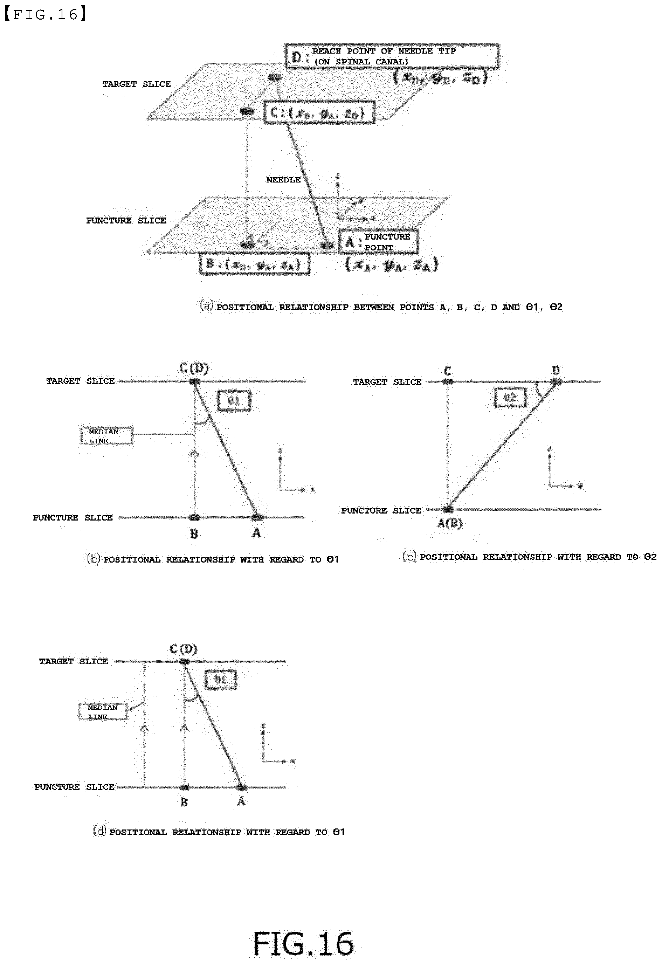

Next, a method of calculating angles in the step S21 of FIG. 9 will be described in detail. Each of FIGs. 15 and 16 is a schematic diagram for explaining a method of calculating a first angle and a second angle.

The control unit calculates three-dimensional coordinate values (mm) of the puncture point A and the target point D. This is done by plotting coordinate values (pix) in a slice plane of the puncture point A and a slice plane of the target point D, a pix-mm ratio of the puncture slice image, a pix-mm ratio of the target slice image and an interval (mm) between the puncture slice image and the target slice image on the three-dimensional coordinates of x, y and z. The origin of the three-dimensional coordinates is set to a point (first point) B corresponding to the median line in the puncture slice image. Then, as shown in FIG. 15, the point B on the skin where the median line and the puncture slice image intersect with each other, the point (second point) C on the skin where the median line and the target slice image intersect with each other, the first angle $\theta 1$ (zBCA) and the second angle $\theta 2$ (zCDA) are set. Then, the control unit calculates the first angle $\theta 1$ and the second angle $\theta 2$ as the puncturing angles of the puncture needle.

First, the control unit calculates $\cos\theta 1$ and $\cos\theta 2$ by vector calculation. With regard to $\cos\theta 1$,

$$\text{If } \vec{a} = \overrightarrow{CA}, \ \vec{b} = \overrightarrow{CB}, \quad \cos\theta 1 = \frac{\vec{a}\cdot\vec{b}}{|\vec{a}||\vec{b}|}$$

$$\text{where } \overrightarrow{CA} = (x_A - x_D, \ y_A - y_A, \ z_A - z_D) = (x_A - x_D, 0, z_A - z_D)$$

$$\overrightarrow{CB} = (x_D - x_D, \ y_A - y_A, \ z_A - z_D) = (0, 0, z_A - z_D) \quad \text{and thus}$$

$$\cos\theta 1 = \frac{(z_A - z_D)^2}{\sqrt{(x_A - x_D)^2 + (z_A - z_D)^2}\sqrt{(z_A - z_D)^2}}$$

$$= \frac{z_A - z_D}{\sqrt{(x_A - x_D)^2 + (z_A - z_D)^2}}$$

With regard to $\cos\theta 2$,

$$\text{If } \vec{c} = \overrightarrow{DC}, \ \vec{d} = \overrightarrow{DA}, \quad \cos\theta 2 = \frac{\vec{c}\cdot\vec{d}}{|\vec{c}||\vec{d}|}$$

$$\text{where } \overrightarrow{DC} = (x_D - x_D, \ y_A - y_D, \ z_D - z_D) = (0, \ y_A - y_D, 0)$$

$$\overrightarrow{DA} = (x_A - x_D, \ y_A - y_D, \ z_A - z_D) \quad \text{and thus}$$

$$\cos\theta 2 = \frac{(y_A - y_D)^2}{\sqrt{(y_A - y_D)^2}\sqrt{(x_A - x_D)^2 + (y_A - y_D)^2 + (z_A - z_D)^2}}$$

$$= \frac{y_A - y_D}{\sqrt{(x_A - x_D)^2 + (y_A - y_D)^2 + (z_A - z_D)^2}}$$

$\theta 1$ and $\theta 2$ are calculated by using the inverse function of COS function with respect to $\cos\theta 1$ and $\cos\theta 2$ calculated in this way.

$$\theta 1 = \cos^{-1}(BC/AC)$$

$$\theta 2 = \cos^{-1}(CD/AD)$$

The first angle $\theta 1$ and the second angle $\theta 2$ calculated in the above step S21 can be optimized to obtain more suitable puncturing angles. As a pre-process, in the puncture slice image, the target slice image and the plurality of slice images therebetween, the control unit identifies a medial limit point (a contralateral limit point which is beyond a body axis in a craniocaudal direction when viewed from the puncture point) and a lateral limit point (an ipsilateral limit point which is not beyond the body axis when viewed from the puncture point) of the range to be reached by the tip of the puncture needle along the boundary line between the spinal cord and the subarachnoid space (S22). For example, as shown in FIG. 13, the control unit plots the contralateral limit point (medial limit point) which is beyond the body axis in the craniocaudal direction when viewed from the puncture point and the ipsilateral limit point (lateral limit point) which is not beyond the body axis when viewed from the puncture point in the reachable range of the subarachnoid space along the boundary line between the spinal cord and the subarachnoid space in the target slice image. Thus, the horizontal reachable range is specified in each slice image.

Thereafter, the process proceeds to the step S22A as a first optimization method or the step S23 as a second optimization method.

As the first optimization method, a method performed by obtaining an average value of angles will be described. Specifically, with respect to the first angle $\theta 1$, the control unit determines medial and lateral angles at the respective medial and lateral limit points of the range to be reached by the tip of the puncture needle specified by the step S22. Similarly, with respect to the second angle $\theta 2$, medial and lateral angles at the respective medial and lateral limit points are determined. The number of pairs of medial and lateral angles is equal to the number of the slice images passing between the puncture slice image and the target slice image. The calculation method of the step S21 can be used to calculate these angles. When a virtual target point D is positioned off the median line, the position of each point of ABCD as a reference is corrected as shown in FIG. 16(d), and then the step S21 is applied. Then, for each of the first angle $\theta 1$ and the second angle $\theta 2$, an average value obtained by integrating the medial and lateral angles is calculated, and the average value is determined as the optimal puncturing angle (S22A). For example, an example of applying the method to an actual patient is shown in the following table.

| slice | $\theta 1$ | | $\theta 2$ | |
|---|---|---|---|---|
| | medial | lateral | medial | lateral |
| 160 | 14.7 | 14.0 | 44.1 | 43.7 |
| 161 | 17.0 | 13.5 | 44.5 | 43.1 |
| 162 | 17.3 | 13.0 | 44.2 | 42.4 |
| 163 | 18.3 | 13.2 | 43.6 | 42.2 |
| 164 | 20.1 | 13.1 | 43.4 | 41.3 |
| 165 | 20.1 | 12.5 | 43.0 | 40.2 |
| 166 | 20.1 | 12.4 | 42.1 | 39.6 |
| 167 | 20.1 | 12.6 | 41.7 | 39.1 |
| 168 | 20.9 | 11.6 | 40.9 | 37.4 |
| 169 | 20.5 | 11.5 | 40.4 | 37.0 |
| 170 | 21.3 | 11.3 | 40.1 | 35.9 |
| 171 | 21.3 | 11.1 | 39.3 | 35.0 |
| 172 | 20.9 | 11.3 | 38.5 | 34.5 |
| 173 | 20.1 | 11.6 | 37.7 | 34.0 |
| 174 | 20.1 | 11.8 | 36.7 | 33.4 |
| 175 | 19.7 | 12.6 | 36.1 | 32.8 |
| 176 | 19.2 | 13.4 | 35.2 | 32.8 |
| 177 | 18.2 | 17.7 | 33.7 | 33.6 |

The table shows the medial and lateral angles of each of the first angle $\theta 1$ and the second angle $\theta 2$ in each slice image. The optimal puncturing angle is 16.1 (deg) on average as the first angle $\theta 1$ and 39.0 (deg) on average as the second angle $\theta 2$. At this time, the process proceeds to the step S24, and the guide part and the support base are prepared using the angle information obtained in the step S22A.

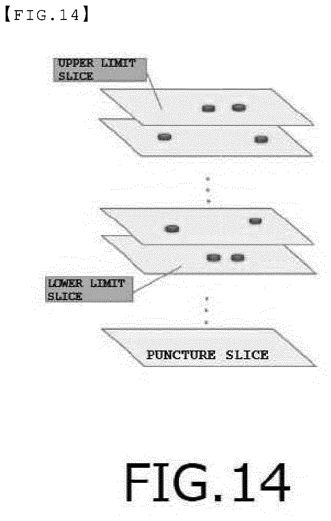

As the second optimization method, a method performed by calculating passing points of a reachable range will be described. After the step S22, as shown in FIG. 14, the control unit specifies vertical distribution of the horizontal limit points (horizontal reachable range) specified in each slice image as the entire reachable range of the puncture needle (S23). Accordingly, the reachable range in which the tip of the puncture needle can safely reach is specified in both the vertical direction and the horizontal direction. As shown in FIG. 14, the reachable range has an ellipse in shape when viewed from a back of a target human body, and has a substantially ellipse body when expressed in three dimensions.

Next, a straight line passing through the puncture point A set in the step S15 and a center of the ellipse or an approximate position thereof is set as an optimum route, and the first angle θ1 and the second angle θ2 are calculated in this setting (S23A). The calculation method of the step S21 can be used as the method of calculating the angles. When the virtual target point D is positioned off the median line, the position of each point of ABCD as the reference is corrected as shown in FIG. 16(d), and then the step S21 is applied. At this time, the process proceeds to the step S24, and the guide part and the support base are prepared using the angle information obtained in the step S23A.

6. Another mode of the method of manufacturing the guide device

In the above description, the target point D set in the step S18 is the center point in the spinal canal, but the target point D that leads to the optimum angle is not limited thereto. That is, the target point D does not need to be within the median plane (plane dividing the human body into two halves), and may be any point within the above-described reachable range. In the above description, the distance of the puncture point A from the median line is 15 mm, but is not limited thereto. The puncture point A may be any point that is the predetermined distance away from the median line. Therefore, as another mode of the method of manufacturing the guide device 1, a method of automatically determining the puncture point A and the target point D will be described. Note that the puncture point A and the target point D may be arbitrarily set on the CT images by the operator as described above.

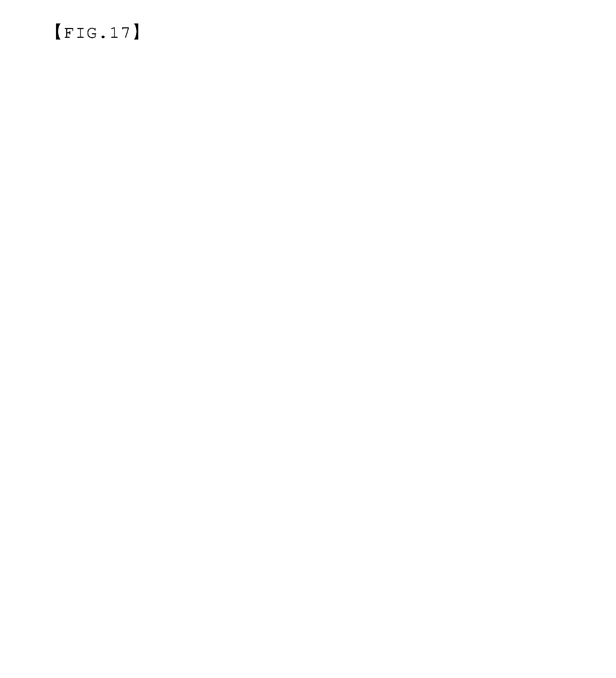

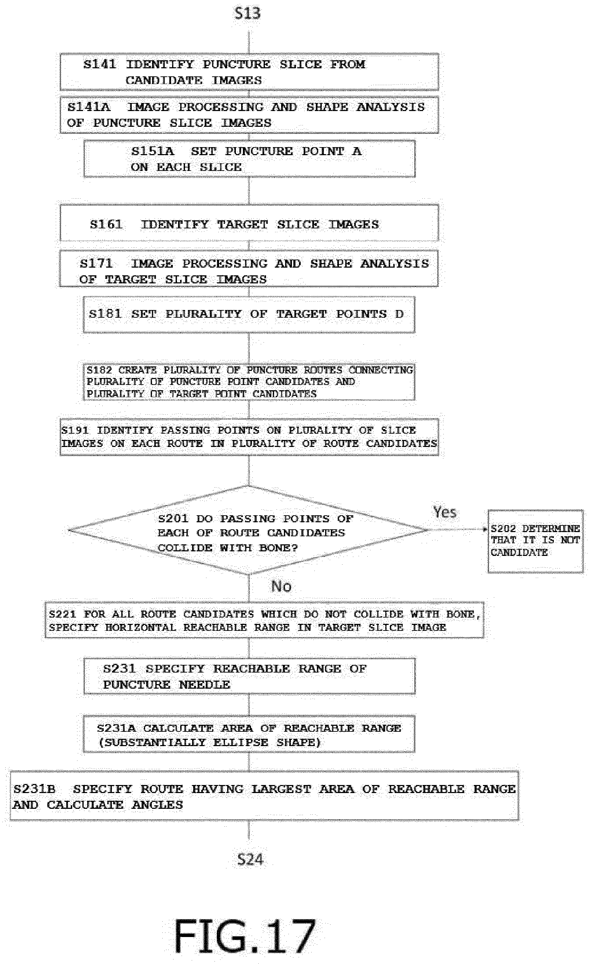

FIG. 17 is a flowchart of a method for automatically determining a puncture point A and a target point D. As shown in FIG. 17, the method includes a step of setting candidates of a plurality of puncture points A on the puncture slice image (S151A), a step of setting candidates of a plurality of target points D on the target slice image (S181), a step of creating a plurality of puncture-route candidates connecting the candidates of the plurality of target points D and the candidates of the plurality of puncture points A (S182), a step of identifying passing points on the plurality of slice images in the plurality of puncture-route candidates (S191), a step of determining whether or not each of the passing points collides with a bone (S201) and a step of determining that a route colliding with the bone is not the candidate (S202). In FIG. 17, the step S141 continues from the step S13 in FIG. 9, and the step S231B continues to the step S24 in FIG. 9. Hereinafter, each step will be described in order.

In the step S141, the control unit identifies all slice images between the slice image including the second lumbar vertebra and the slice image including the fifth lumbar vertebra as the candidates for the puncture slice images (step S141). Note that the slice image is not limited to the slice image including the second lumbar vertebra, and may be a slice image including a lumbar vertebra having a vertebra level higher than that of the second lumbar vertebra. Here, the control unit executes the image processing such as the binarization processing of each of the puncture slice images and the shape analysis thereof, and extracts a contour line of the skin (step S141A).

Next, in each puncture slice image, the control unit sets a plurality of points apart from the first point B by the predetermined distance in the transverse direction (left or right direction) along the contour line. That is, in the step S151A, in the puncture slice image as shown in FIG. 2, the control unit sets the plurality of points as the candidates for the puncture point A at a predetermined interval or at random from the first point B to the left and/or right along the contour line of the skin.

The predetermined interval is not particularly limited, but is preferably 0.1 mm to 0.2 mm. According to such a range, it is possible to reduce the possibility that the puncture needle collides with the bone when inserting the puncture needle, and to set the route from the puncture point A to the target point D more appropriately. The number of candidates for the puncture point A is not particularly limited, but is preferably 1 to 20, and more preferably 1 to 10, per slice image. However, the number of candidates may be more than 100, and may be 500 or more. Such a range increases the number of combinations of the puncture point A and the target point D. For this reason, even if one straight line AD among the plurality of straight lines AD (route candidates) collides with the bone, it can be changed to another straight line AD (route candidate) that does not collide with the bone.

Next, the control unit identifies all slice images between the slice image including the first lumbar vertebra and the slice image including the fourth lumbar vertebra as the candidates for the target slice image (step S161). Note that the slice image is not limited to the slice image including the first lumbar vertebra, and may be a slice image including a lumbar vertebra having a vertebra level higher than that of the first lumbar vertebra. Then, the control unit executes the image processing such as the binarization processing of each of the target slice images and the shape-analysis thereof (step S171). This identifies the spinal canal and the back-side boundary line between the spinal cord and the subarachnoid space in the spinal canal.

Next, the control unit sets the plurality of target points D on the identified boundary line between the spinal cord and the subarachnoid space in the spinal canal (step S181). That is, the control unit sets a plurality of points as candidates of the target point D on the boundary line shown in FIG. 10 at a predetermined interval or at random.

The predetermined interval is not particularly limited, but is preferably 0.1 mm to 0.2 mm. According to such a range, it is possible to reduce the possibility that the puncture needle collides with the bone when inserting the puncture needle, and to appropriately set the distance from the puncture point A to the target point D. The number of candidates for the target point D is not particularly limited, but is preferably 1 to 20, and preferably 1 to 10. Such a range increases the number of combinations with the puncture point A. For this reason, even if one straight line AD (route candidate) among the plurality of straight lines AD (route candidates) collides with the bone, it can be changed to another straight line AD that does not collide with the bone.

Next, the control unit creates a plurality of straight line AD groups (hereinafter referred to as "route candidates") connecting the plurality of candidates for the puncture point A and the plurality of candidates for the target point D (S182). The number of route candidates may be the number of 1:1 combinations of the puncture point A and the target point D, or the number of all possible combinations of the puncture point A and the target point D. Further, each of the route candidates is a combination in which the candidate of the puncture point A is identified on the same image as the slice image of the candidate of the target point D or on the slice image between the two lumbar vertebrae that are lower (closer to the feet) than the slice image of the target point D. In the latter case, as a preferable combination, the candidate of the puncture point A is identified on a slice image between a lumbar vertebra immediately below the position of the slice image of the target point D and a lumbar vertebra further below and next to the lumbar vertebra. For example, if the puncture point A is on a slice image between the third lumbar vertebra and the fourth lumbar vertebra, the target point D is identified on a slice image between the first lumbar vertebra and the fourth lumbar vertebra, preferably on a slice image between the second lumbar vertebra and the third lumbar vertebra.

Next, the control unit identifies passing points on the plurality of slice images through which each of the set route candidates passes (S191). That is, as shown in FIG. 11, in the plurality of slice images between the puncture slice image including the puncture point A and the target slice image including the target point D, coordinates of each of the passing points as the route candidate are calculated. The control unit executes this calculation for all the route candidates. The coordinates of the passing point are calculated as described in the step S19.

Next, the control unit determines whether or not the passing points collide with the bone in each of the plurality of route candidates (step S201). The determination is performed as described in the step S20. Then, the control unit determines whether or not all the route candidates collide with the bone. If there is a route candidate which collides with the bone, it is determined that the route candidate is not the candidate (S202). If there is a route candidate which does not collide with the bone, the process proceeds to step S221.

Next, for all the route candidates which do not collide with the bone, the control unit identifies the medial limit point (the contralateral limit point which is beyond the body axis in the craniocaudal direction when viewed from the puncture point) and the lateral limit point (the ipsilateral limit point which is not beyond the body axis when viewed from the puncture point) of the range to be reached along the boundary line between the spinal cord and the subarachnoid space in the plurality of slice images between the puncture point A and the target point D of each route candidate (S221). For example, as shown in FIG. 13, the control unit plots the contralateral limit point (medial limit point) which is beyond the body axis in the craniocaudal direction when viewed from the puncture point and the ipsilateral limit point (lateral limit point) which is not beyond the body axis when viewed from the puncture point in the reachable range of the subarachnoid space along the boundary line between the spinal cord and the subarachnoid space in the target slice image. Thus, the horizontal reachable range is specified in each slice image.

Next, for each of all the route candidates, as shown in FIG. 14, the control unit specifies the vertical distribution of the horizontal limit points (horizontal reachable range) specified in each slice image as the entire reachable range of the puncture needle (S231). Accordingly, the reachable range in which the tip of the puncture needle can safely reach is specified in both the vertical direction and the horizontal direction. As shown in FIG. 14, the reachable range has an ellipse in shape when viewed from the back of the target human body. Further, the reachable range has a substantially ellipse body when expressed in three dimensions. For each of all the route candidates, the reachable range having such an elliptical shape is specified.

The control unit calculates an area of the reachable range specified by the step S231 for each of all the route candidates (S231A).

Next, the control unit compares each area calculated in the step S231A with each other, and specifies a route indicating the reachable range having the largest area. Using the puncture point A and the target point D of the specified route, the first angle $\theta1$ and the second angle $\theta2$ of the route are calculated (S231B). For the calculation thereof, the method of the step S21 is used. When the target point D is positioned off the median line, the position of each point of ABCD as the reference is corrected as shown in FIG. 16(d), and then the step S21 is applied. Then, the process proceeds to the step S24. The angle data calculated in the step S231B is used for the first angle $\theta1$ and the second angle $\theta2$ used in the step S24.

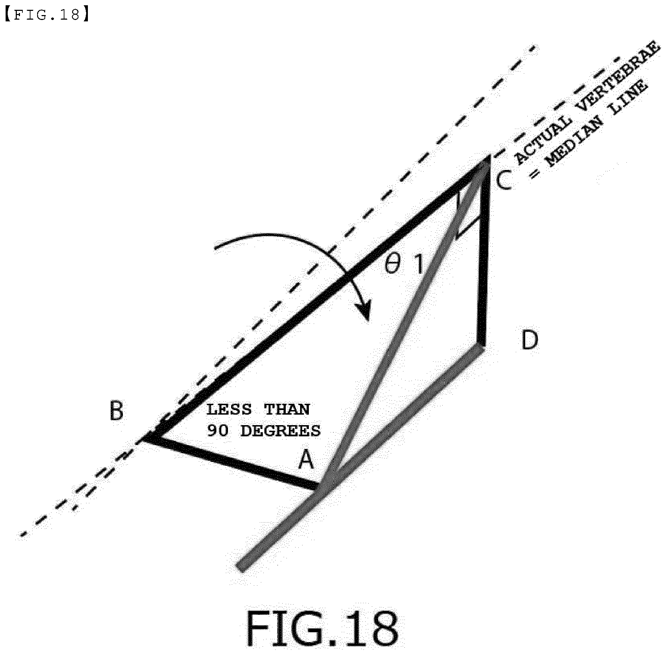

When a patient is in a lateral decubitus position, the spine may bend downward (toward the bed). In this case, the second point C moves downward and the $\angle ABC$ is less than 90°. FIG. 18 is a schematic diagram illustrating another configuration example for explaining the method of calculating the first angle and the second angle. As shown in FIG. 18, when the spine bends, CT images are taken in the lateral decubitus position. Next, the control unit calculates the first angle $\theta1$ and the second angle $\theta2$ using the method of S21. As a result, the first angle $\theta1$ is generally calculated to be less than 30°, and the second angle $\theta2$ is generally calculated to be less than 30°. This makes it possible to obtain the first angle $\theta1$ being slightly larger than the first angle $\theta1$ which is obtained without considering the bend of the spine. In this regard, a marker can be attached to each of the points A, B and C in order to align the images with a body surface of the patient at the time of photographing, which contributes to improving the accuracy of puncturing at the time of surgery.

As described above, according to the method of manufacturing the guide device of the present invention, the first angle $\theta1$ and the second angle $\theta2$ can be optimally adjusted. This makes it possible to provide the guide device 1 which is adapted to the body shape and the skeleton of each patient. In addition, it is possible to provide the guide device 1 which reliably avoids interference of the bone structure by specifying the puncture point A and calculating the puncturing angles. Therefore, it is possible to support a procedure that does not rely on the sense or the experience of the operator.

<<2nd embodiment>>

Next, a second embodiment of the guide device of the present invention will be described with reference to the accompanying drawing.

Hereinafter, the second embodiment will be described, but differences from the first embodiment described above will be mainly described, and descriptions of the same matters will be omitted. FIG. 19 is a perspective view schematically showing a guide device according to a second embodiment of the present invention.

A guide device 10 of the present embodiment is the same as the guide device 1 of the first embodiment except that a configuration of the guide part 2 is different. Specifically, the guide part 2 of the guide device 10 of the present embodiment is composed of the main body 21 and the guide rail 22, and does not include the grip portion 23. Therefore, the guide part 2 is formed into a triangular shape. With such a configuration, the configuration of the guide part 2 can be made compact.

The main body 21 of the present embodiment does not have the second support portion 213. Therefore, the guide rail 22 is provided so as to connect the upper end of the first support portion 212 of the main body 21 and the reinforcing portion 211. The length of the reinforcing portion 211 is not particularly limited, but may be shorter than the length of the reinforcing portion 211 of the first embodiment. In addition, the length of the first support portion 212 is longer than the length of the first support portion 212 of the first embodiment. With such a configuration, the length of the guide rail 22 is longer than the length of the guide rail 22 of the first embodiment. Therefore, the entire height of the main body 21 from the support base 3 is increased.

The distal end of the guide rail 22 is connected to the reinforcing portion 211. That is, the distal end of the guide rail 22 is spaced from the notch 33 by a predetermined distance toward the first support portion 212 along the lengthways direction of the reinforcing portion 211. Thus, even if the member for fixing the puncture needle 11 is attached to the guide rail 22, the puncture needle 11 can be reliably passed through the notch 33.

The guide device 10 of the present embodiment includes the fixing member 9 for fixing the puncture needle 11. The fixing member 9 is attached to the guide rail 22 and is movable along the guide rail 22 without separation. The fixing member 9 has two clamping members, each of which has a "U" shape in a plan view. As shown in FIG. 19, the two clamping members are arranged side by side in the vertical direction, and clamp a plate member attached to the puncture needle 11. Thus, the puncture needle 11 can be attached to the fixing member 9. The shape of each clamping member varies depending on the shape of the plate member attached to the puncture needle 11.

With such a configuration, since the guide rail 22 is long, the guide device 10 of the present embodiment can guide the puncturing for a long distance (period) even after the puncture needle 11 is inserted into the patient's body. In addition, since the main body 21 is high, the guide device 10 of the present embodiment is excellent in holding property for the operator. Therefore, the guide device 10 of the present embodiment is suitable for an inexperienced operator and a patient having a long distance from the puncture point to the target point (for example, an obese patient). In addition, the guide device 10 of the present embodiment has the same effects as those of the guide device 1 of the first embodiment.

<<3rd embodiment >>

Next, a third embodiment of the guide device of the present invention will be described.

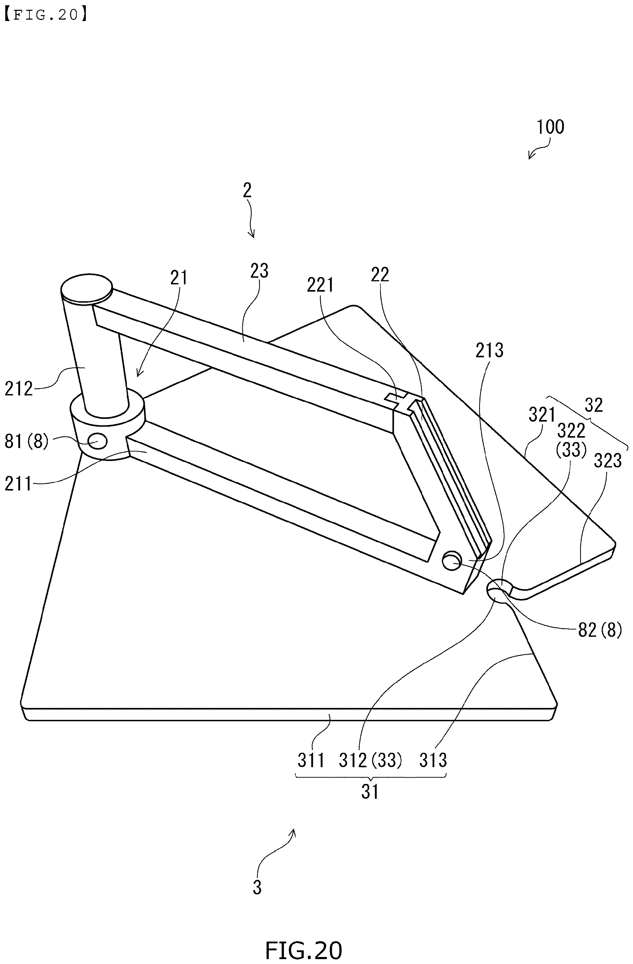

Hereinafter, the third embodiment will be described, but differences from the first embodiment described above will be mainly described, and descriptions of the same matters will be omitted. FIG. 20 is a perspective view schematically showing a guide device according to a third embodiment of the present invention. Note that the same components as those in the first embodiment described above are denoted by the same reference numerals.

A guide device 100 of the present embodiment is different from the guide device 1 of the first embodiment in that the guide device 100 includes an angle changing device 8. The angle changing device (second angle changing device) 8 is provided in the guide part 2, and can change the second angle $\theta2$. The angle changing device 8 includes an adjusting portion 81 that can adjust the length of the first support portion 212, and a pivot portion 82 that rotates the guide rail 22. Specifically, the adjusting portion 81 is provided at a lower portion of the first support portion 212 of the main body 21, and is composed of, for example, a screw. The length of the first support portion 212 can be changed by operating the adjusting portion 81. The pivot portion 82 is provided at an upper portion of the second support portion 213, and is composed of, for example, a rotation shaft. The guide rail 22 is configured to be rotatable about the pivot portion 82 at its distal end. It is preferable to locate the pivot portion 82 as low as possible on the second support portion 213. Even if an inclination angle $\theta 2$ of the guide rail 22 changes, the guide device 100 is configured to allow the puncture needle to always pass through the puncture point A at the notch.

Further, the guide rail 22 has a fitting portion 221 at its proximal end, and is configured to be detachable from the grip portion 23 by the fitting portion 221. That is, the guide rail 22 is not fixed to the grip portion 23, and is fitted to the grip portion 23 by the fitting portion 221. Thus, for example, when the height of the first support portion 212 is lowered by the adjusting portion 81, the grip portion 23 approaches the reinforcing portion 211 while the fitting portion 221 follows the grip portion 23. For this reason, the incline of the guide rail 22 becomes gentler. In other words, the first support portion 212 is lowered by the operation of the adjusting portion 81, thereby reducing the angle of the guide rail 22 with respect to the support base 3. As described above, the guide device 100 of the present embodiment can change the angle of the guide rail 22.

The adjusting portion 81 may be provided at the distal end of the grip portion 23. This makes it possible to change the length of the grip portion 23. For example, when the length of the grip portion 23 is shortened, the proximal end of the guide rail 22 follows the distal end of the grip portion 23. As a result, the incline of the guide rail 22 becomes gentler. Therefore, the angle of the guide rail 22 with respect to the support base 3 is reduced. Conversely, when the length of the grip portion 23 is increased, the fitting portion 221 follows the distal end of the grip portion 23. As a result, the incline of the guide rail 22 becomes steeper. Therefore, the angle of the guide rail 22 with respect to the support base 3 is increased. In this way, the adjusting portion 81 can change the angle of the guide rail 22 with respect to the support base 3.

The angle of the puncture needle with respect to the surface of the human body (the angle $\theta 2$ of the puncture needle with respect to the line perpendicular to the surface of the patient's body) is slightly different for each patient. Therefore, according to the guide device 100 of the present embodiment, it is possible to puncture the target point D with the puncture needle reliably and accurately for any patient by changing the angle of the guide rail 22 with the adjusting portion 81. That is, the guide device 100 of the present embodiment can be adapted individually to the puncturing procedures for a wide variety of patients. In addition, the guide device 100 of the present embodiment has the same effects as those of the guide device 1 of the first embodiment.

The guide device 100 of the present embodiment can change the angle (first angle $\theta 1$) at which the extension line L1 of the reference portion 321 (311) intersects with the imaginary line L2 passing through the notch 33 (FIG. 5). This is performed, for example, by changing (lengthening or shortening) the length of the puncture guide portion 313 to create the support base 3. Further, the first angle $\theta 1$ can be changed by changing $\angle ABC$ in FIG. 5 to create the support base 3. Furthermore, the first angle $\theta 1$ can be changed by changing the position of the skin point B, the position of the target point D or the like.

<<4th embodiment >>

Next, a fourth embodiment of the guide device of the present invention will be described. The fourth embodiment will be described below, but differences from the first embodiment described above will be mainly described, and descriptions of the same matters will be omitted. Note that the same components as those in the first embodiment described above are denoted by the same reference numerals.

A guide device 1 of the present embodiment is the same as the guide device 1 of the first embodiment except that the guide device 1 does not include the second blade portion 32. Specifically, the guide device 1 of the fourth embodiment includes only the first blade portion 31. Therefore, the entire size of the guide device 1 can be made compact, which is advantageous in terms of portability and storage. In addition, the guide device 1 of the present embodiment has the same effects as those of the guide device 1 of the first embodiment.

<<5th embodiment >>

Next, a fifth embodiment of the guide device of the present invention will be described.

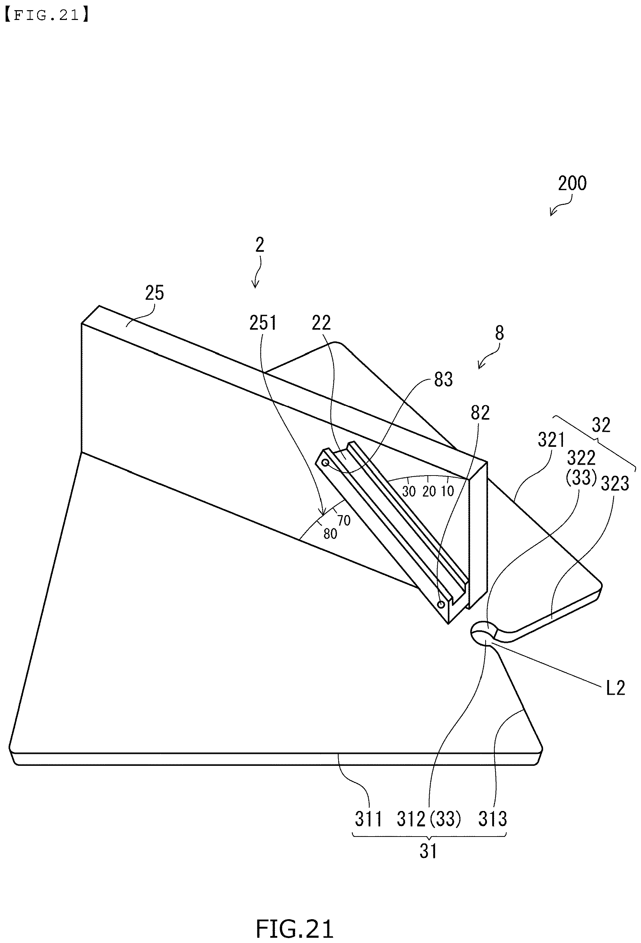

Hereinafter, the fifth embodiment will be described, but differences from the first embodiment and the third embodiment described above will be mainly described, and descriptions of the same matters will be omitted. FIG. 21 is a perspective view schematically showing a guide device according to a fifth embodiment of the present invention. Note that the same components as those in the first embodiment and the third embodiment described above are denoted by the same reference numerals.

A guide device 200 of the present embodiment is different from the guide device 1 of the first embodiment in that the guide part 2 is composed of a plate-like member 25 instead of the main body 21 and the grip portion 23. The plate-like member 25 has a function of fixing the guide rail 22. The plate-like member 25 is provided perpendicularly to the support base 3 along the imaginary line L2. Further, the plate-like member 25 is positioned to move in parallel from the central portion of the support base 3 toward the second blade portion 32 by the width of the guide rail 22. A scale 251 indicating an angle of the guide rail 22 is displayed on one side surface of the plate-like member 25. The scale 251 includes 0°, that is a line coinciding with a line perpendicular to the support base 3, 90°, that is a line coinciding with a line horizontal to the support base 3, and 10° to 80° therebetween. Accordingly, the guide rail 22 can be easily set to the desired second angle θ2.

The guide device 200 of the present embodiment is different from the guide device 1 of the first embodiment in that the guide device 200 includes an angle changing device (second angle changing device) 8. The angle changing device 8 is provided in the guide part 2, and can change the second angle θ2. The angle changing device 8 includes the pivot portion 82 for rotating the guide rail 22 and a fixing portion 83.

The pivot portion 82 has a function of retaining the guide rail 22 to the plate-like member 25. The pivot portion 82 is provided at the distal end of the guide rail 22, and is composed of a rotation shaft. The guide rail 22 is provided rotatably around the pivot portion 82 at the distal end of the guide rail 22. The fixing portion 83 has a function of fixing the guide rail 22 to the plate-like member 25. The fixing portion 83 is provided at the proximal end of the guide rail 22 and is composed of, for example, a screw. The fixing portion 83 is released to rotate the guide rail 22. This makes it possible to change the second angle θ2 of the guide rail 22 to the desired second angle θ2 when viewed from the puncture point A at the center of the notch 312.

The guide rail 22 is retained to the one side surface of the plate-like member 25 by the pivot portion 82. In this state, the guide rail 22 can be set to the desired second angle θ2 according to the scale 251. The fixing portion 83 fixes the guide rail 22 to the plate-like member 25. As a result, the second angle θ2 can be easily and quickly changed to obtain the guide device 200 having the desired second angle θ2.

The plate-like member 25 may be positioned to move in parallel by the width of the guide rail 22 from the central portion of the support base 3 toward the first blade portion 31, that is, toward a direction opposite to the direction of paragraph 0123. In this case, the guide rail 22 and the scale 251 are provided on the other side surface opposite to the side surface described above of the plate-like member 25.

With such a configuration, since the second angle θ2 of the guide rail 22 can be easily changed, the guide device 200 of the present embodiment can exhibit the same effects as those of the guide device 100 of the third embodiment. In addition, the guide device 200 of the present embodiment has the same effects as those of the guide device 1 of the first embodiment.

(Modified example)

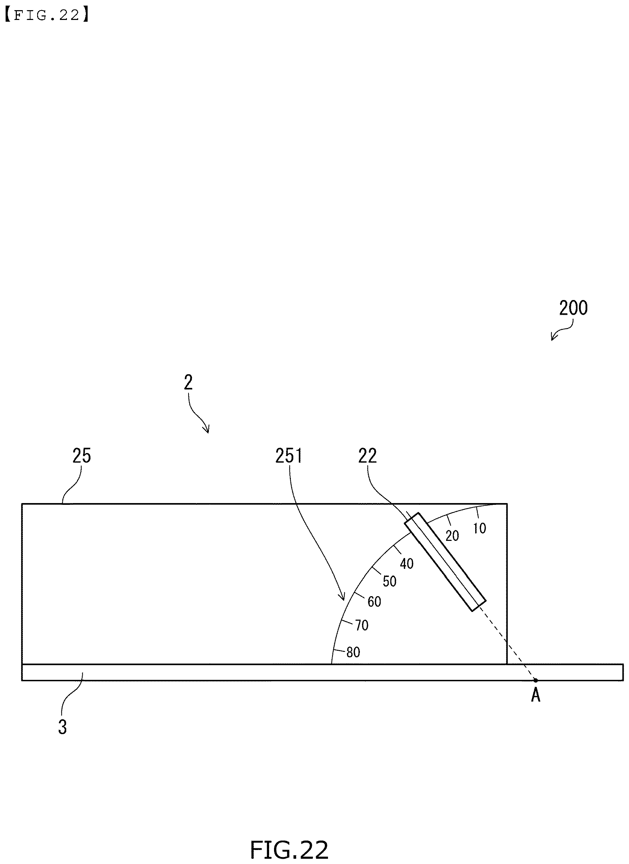

Next, a modified example of the fifth embodiment of the guide device of the present invention will be described. FIG. 22 is a side view schematically showing a modified example of the guide device according to the fifth embodiment of the present invention. Note that the same components as those in the fifth embodiment described above are denoted by the same reference numerals.

The modified example of the guide device 200 of the present embodiment is different from the guide device 200 of the fifth embodiment described above in that the guide device 200 does not include the pivot portion 82. That is, the guide rail 22 is configured to be rotatable about the puncture point A. Therefore, the scale 251 has numerical values of an angle which is defined from a line perpendicular to the support base 3 and is centered on the puncture point A, that is, the second angle θ2 about the target point D. In FIG. 22, the numerical values are displayed at intervals of 10°, but the numerical values may be displayed at intervals of 5°.

After determining the second angle θ2 about the target point D, the angle of the scale 251 is set to the second angle θ2. Then, the guide rail 22 is fixed to the plate-like member 25 by the fixing member (not shown) such as the screw. At this time, the guide rail 22 may be movable in the vertical direction. The guide rail 22 is preferably provided so that a gap is formed between the guide rail 22 and the skin of the patient.

The modified example having such a configuration also has the same effects as those of the guide device 1 of the first embodiment, the guide device 100 of the third embodiment and the guide device 200 of the fifth embodiment described above.

<<6th embodiment >>

Next, a sixth embodiment of the guide device of the present invention will be described.

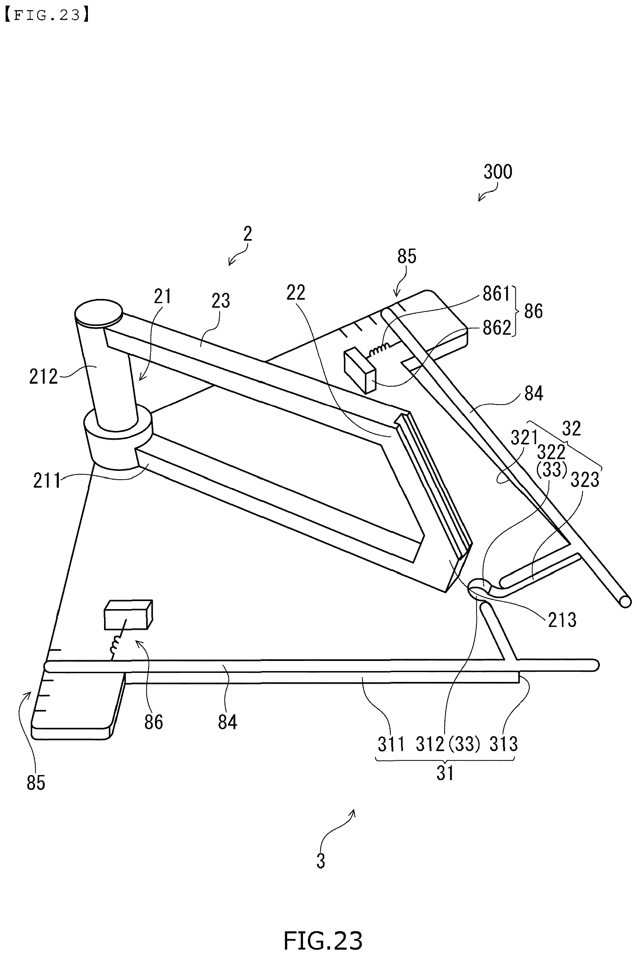

Hereinafter, the sixth embodiment will be described, but differences from the first embodiment described above will be mainly described, and descriptions of the same matters will be omitted. FIG. 23 is a perspective view schematically showing a guide device according to a sixth embodiment of the present invention. Note that the same components as those in the first embodiment described above are denoted by the same reference numerals.

A guide device 300 of the present embodiment is different from the guide device 1 of the first embodiment in that the guide device 300 includes angle changing devices (first angle changing devices) 8. Specifically, as shown in FIG. 23, the angle changing devices 8 are provided in the support base 3, and can change the first angle θ1. The angle changing devices 8 are respectively located on a side of the first blade portion 31 and on a side of the second blade portion 32 in the guide device 300 of the present embodiment. A configuration of the angle changing device 8 on the side of the first blade portion 31 is the same as that of the angle changing device 8 on the side of the second blade portion 32. Thus, the angle changing device 8 on side of the second blade portion 32 will be described as a representative. The angle changing device 8 includes a variation reference portion 84, a scale portion 85 and a pressure portion 86.

The variation reference portion 84 has the same function as that of the reference portion 321. The variation reference portion 84 is provided on the support base 3 along the reference portion 321 and the puncture guide portion 323, and is formed into a T-shape. However, the shape of the variation reference portion 84 is not limited thereto. The variation reference portion 84 is composed of an elongated reference bar and a rotation bar. The reference bar is orthogonal to the rotation bar.

A distal end of the rotation bar of the variation reference portion 84 is connected at the vicinity of the notch 33 such that the variation reference portion 84 is configured to be rotatable about the distal end of the rotation bar. Since the variation reference portion 84 is formed into the T-shape, the reference bar and the rotation bar are always orthogonal to each other. Therefore, even if the variation reference portion 84 is rotated, it is possible to reliably indicate an appropriate puncture point A. The length of the rotation bar corresponds to the length of the puncture guide portion 313. Specifically, the length of the rotation bar is preferably about 3 mm to 20 mm, more preferably about 5 mm to 18 mm and most preferably 15 mm. This makes it possible to indicate the puncture point A suitable for the puncturing.

Further, a proximal end (rear end) of the variation reference portion 84 has a fastener such as a hook (not shown). Thus, the variation reference portion 84 can be retained to the scale portion 85 at the rear end thereof. When the guide device 300 of the present embodiment is used for a patient, the guide device 300 is located in the waist of the patient so as to align the variation reference portion 84 with the median line of the patient.

The scale portion 85 is formed so as to protrude outward from the second blade portion 32 in the vicinity of the rear end of the reference portion 321. A scale indicating the first angle θ1 is displayed on a surface of the scale portion 85. Thus, the first angle θ1 can be easily recognized. In this regard, the scale portion 85 has a receiving portion (not shown) for being joined with the fastener of the variation reference portion 84 on a side surface or a back surface thereof. This allows the variation reference portion 84 to be fixed to the receiving portion at various angles.

The pressure portion 86 has a function of pressing the variation reference portion 84, and is provided on the support base 3. The pressure portion 86 includes pressure means 861 formed of a spring or the like, and fixing means 862 for fixing the pressure means 861. The pressure means 861 is coupled to the variation reference portion 84. This allows the variation reference portion 84 to be pressed, thereby accurately maintaining the first angle θ1. The fixing means 862 is not particularly limited as long as the pressure means 861 can be fixed thereto. The main body 21 and the reinforcing portion 211 of the guide part 2 may serve as the fixing means 862.

With such a configuration, the angle of the reference bar of the variation reference portion 84 with respect to the imaginary line L2, that is, the first angle θ1, can be easily changed. This makes it possible to perform an appropriate puncturing procedure suitable for the body shape of each patient. In addition, the guide device 300 of the present embodiment can exhibit the same effects as those of the guide device 1 of the first embodiment and the guide device 100 of the third embodiment.

(Modified example)

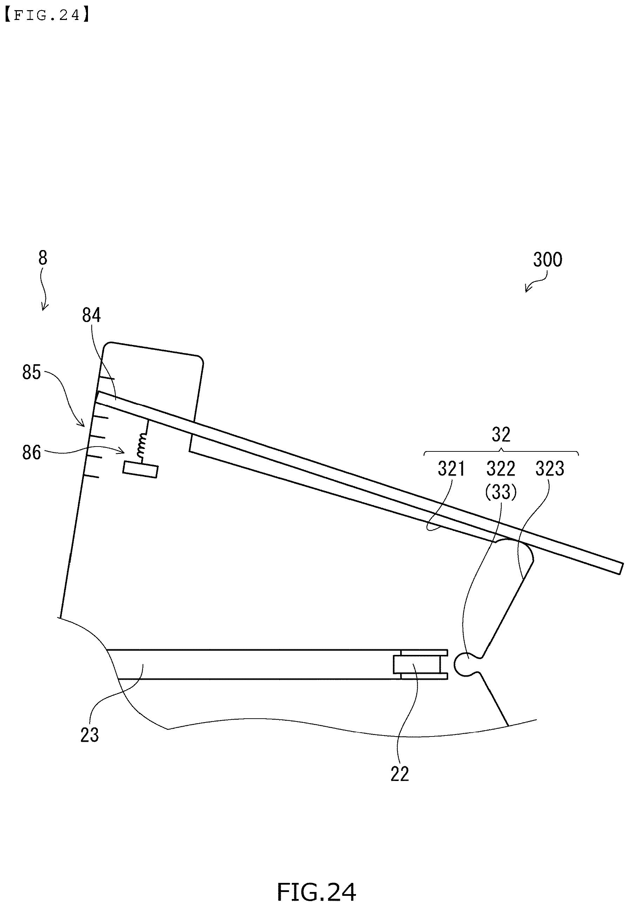

Next, a modified example of the sixth embodiment of the guide device of the present invention will be described. FIG. 24 is a top view schematically showing a modified example of the guide device according to the sixth embodiment of the present invention. Note that the same components as those in the sixth embodiment described above are denoted by the same reference numerals.

The modified example of the guide device 300 of the present embodiment is different from the guide device 300 of the sixth embodiment described above in terms of the shape of the variation reference portion 84 and the configuration of the puncture guide portion 323. That is, the variation reference portion 84 is formed into a rod shape. The puncture guide portion 323 is rotatable about the notch 33 (puncture point A). Thus, a portion of the second blade portion 32 near the puncture guide 323 is retractable or can be accommodated within the second blade portion 32. As a result, the puncture guide portion 323 can perform a circular motion with the length of the puncture guide portion 323 as a radius around the notch 33.

The variation reference portion 84 is always in contact with the puncture guide portion 323 so as to be orthogonal to the puncture guide portion 323. This contact point becomes the first point B. When the puncture guide portion 323 is rotated, a position of the proximal end of the variation reference portion 84 is adjusted so that the variation reference portion 84 is always orthogonal to the puncture guide portion 323. Specifically, when the puncture guide portion 323 is rotated counterclockwise, the proximal end of the variation reference portion 84 is retained at a position below the scale portion 85 shown in FIG. 24 in order to maintain an orthogonal relationship between the variation reference portion 84 and the puncture guide portion 323. As a result, the variation reference portion 84 and the puncture guide portion 323 are always orthogonal to each other. This makes it possible to reliably perform the puncturing through the appropriate puncture point A.

The modified example having such a configuration also has the same effects as those of the guide device 1 of the first embodiment, the guide device 100 of the third embodiment and the guide device 200 of the fifth embodiment described above.

<<7th embodiment>>

Next, a seventh embodiment of the guide device of the present invention will be described.

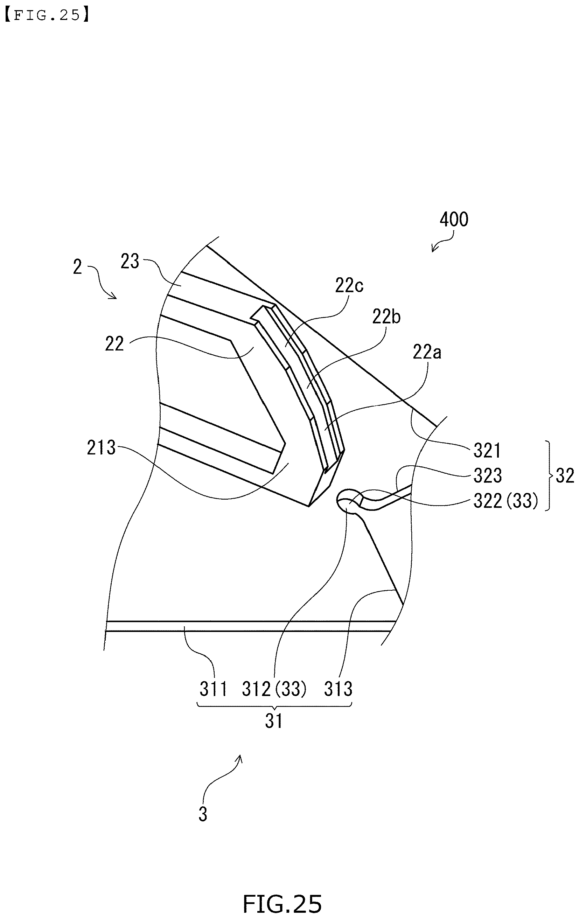

The seventh embodiment will be described below, but differences from the first embodiment described above will be mainly described, and descriptions of the same matters will be omitted. FIG. 25 is a perspective view schematically showing a guide device according to a seventh embodiment of the present invention. Note that the same components as those in the first embodiment described above are denoted by the same reference numerals.

A guide device 400 of the present embodiment is different from the guide device 1 of the first embodiment in that the guide device 400 includes angle changing devices (second angle changing devices) 8. That is, the guide device 400 is different from the guide device 1 of the first embodiment in that the guide rail 22 is bent at a plurality of angles. Specifically, the guide rail 22 also has the function of the angle changing devices 8 for changing the second angle $\theta 2$. The guide rail 22 is composed of a plurality of sub-rails set at the plurality of angles with respect to the perpendicular line. For example, as shown in FIG. 25, the guide rail 22 has a first sub-rail 22a, a second sub-rail 22b and a third sub-rail 22c from the distal end of the guide rail 22.

For example, the first sub-rail 22a is set at 25° with respect to the line perpendicular to the support base 3. The second sub-rail 22b is set at 30° with respect to the line perpendicular to the support base 3. The third sub-rail 22c is set at 35° with respect to the line perpendicular to the support base 3. This allows one guide device 400 to provide three second angles $\theta 2$. These angles with respect to the perpendicular line is not particularly limited, and can be appropriately set to desired angles.

A bending point between the first sub-rail 22a and the second sub-rail 22b and a bending point between the second sub-rail 22b and the third sub-rail 22c function as the angle-changing devices 8. The number of sub-rails is not limited to three, and may be four or more. Further, when the second sub-rail 22b or the third sub-rail 22c is used for the puncturing, the guide part 2 can be moved backward by moving means (not shown). This makes it possible to adjust alignment of the puncture needle to align the puncture point A with the notch 33 or reaching of the puncture needle to the target point D.

With such a configuration, the guide device 400 of the present embodiment can have the plurality of second angles $\theta 2$. This makes it possible to perform an appropriate procedure according to the body shape of each patient. The guide device 400 of the present embodiment has the same effects as those of the guide device 1 of the first embodiment, the guide device 100 of the third embodiment and the guide device 200 of the fifth embodiment.

<<8th Embodiment>>

Next, an eighth embodiment of the guide device of the present invention will be described.

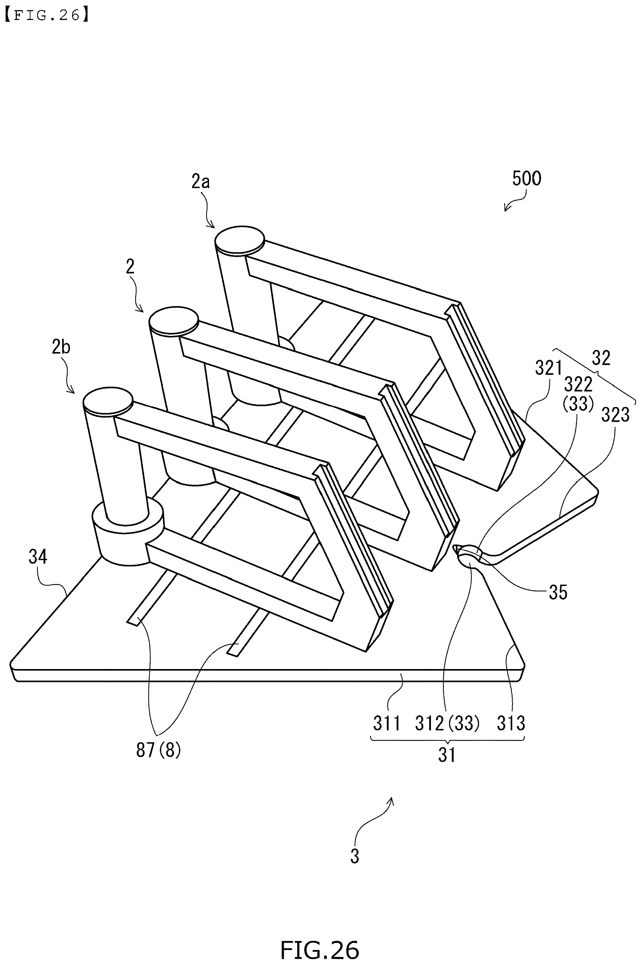

The eighth embodiment will be described below, but differences from the first embodiment described above will be mainly described, and descriptions of the same matters will be omitted. FIG. 26 is a perspective view schematically showing a guide device according to an eighth embodiment of the present invention. Note that the same components as those in the first embodiment described above are denoted by the same reference numerals.

A guide device 500 of the present embodiment is different from the guide device 1 of the first embodiment in that the guide part 2 includes a plurality of guide parts and the guide device 500 includes an angle changing device (second angle changing device) 8. Specifically, as shown in FIG. 26, the guide part 2 includes the guide part 2 described in the first embodiment and two sub-guide parts 2a, 2b. Each of the sub-guide parts 2a, 2b has the same function and configuration as the guide part 2 except that the second angle $\theta 2$ differs. Therefore, a detailed description thereof will be omitted.

The second angle $\theta 2$ of the sub-guide part 2a is set, for example, at 25° with respect to the line perpendicular to the support base 3. Further, the second angle $\theta 2$ of the guide part 2 is set, for example, at 30° with respect to the line perpendicular to the support base 3. Furthermore, the second angle $\theta 2$ of the sub-guide part 2b is set, for example, at 35° with respect to the line perpendicular to the support base 3. This allows one guide device 500 to provide three second angles $\theta 2$. These angles with respect to the perpendicular line are not particularly limited, and can be appropriately set to the desired angles. The guide part 2 may have three or more sub-guide parts. In this case, the length of each of the reinforcing portions 211 of the guide part 2 and the sub-guide parts 2a, 2b may be adjusted so that the puncture point A is aligned with the notch 33 in accordance with each set second angle θ2.

The guide part 2, the sub-guide part 2a and the sub-guide part 2b are provided parallel to each other with a predetermined distance therebetween. However, the predetermined distance is not particularly limited thereto as long as the guide parts 2 and the sub-guide parts 2a, 2b are provided on the support base 3.

As shown in FIG. 26, the angle changing device 8 is provided on the support base 3 and has a function of changing the second angle θ2. The angle changing device 8 includes a rail portion 87 and fixing means (not shown).

The rail portion 87 has a function of moving the guide part 2 and the sub-guide parts 2a, 2b. The rail portion 87 includes two rails. The rail portion 87 is provided on the support base 3 along a direction orthogonal to the guide part 2. Thus, the guide part 2 and the sub-guide parts 2a, 2b can be moved along the rail portion 87, enabling to use one guide part having an appropriate second angle θ2 for the puncturing with the puncture needle. The two rails of the rail portion 87 are arranged at a predetermined interval. The interval is not particularly limited. The number of rails is not particularly limited, and may be one.

The fixing means has a function of fixing the guide part 2 and the sub-guide parts 2a, 2b to the support base 3. The fixing means is provided on the back or rear surface of the support base 3, and is composed of a screw or the like. The fixing means is released, allowing the guide part 2 and the sub-guide parts 2a, 2b to move on the rail portion 87.

The support base 3 of the guide device 500 of the present embodiment is formed into a pentagon shape similar to a trapezoidal shape. Therefore, a base portion 34 of the support base 3 is formed parallel to the rail portion 87. Therefore, lower proximal ends of the guide part 2 and the sub-guide parts 2a, 2b are configured to move along the base portion 34 and to be fixed by the fixing means.

Further, the support base 3 has a marker 35 near the notch 33. The marker 35 functions as positioning means for aligning each of the guide part 2 and the sub-guide parts 2a, 2b with the notch 33 so that the puncture needle can reach the puncture point A through the notch 33. Each of the guide part 2 and the sub-guide parts 2a, 2b can be positioned at an appropriate position on the support base 3 by the marker 35. This makes it possible to allow the puncture needle to reliably reach the puncture point A.

In this way, any one of the guide part 2 and the sub-guide parts 2a, 2b having the second angle θ2 suitable for the patient can be moved and fixed to the support base 3, aligning it with the marker 35. Accordingly, the guide device 500 of the present embodiment enables the puncturing so as to correspond to the plurality of second angles θ2. Therefore, an appropriate procedure can be performed according to the body shape of each patient. The guide device 500 of the present embodiment has the same effects as those of the guide device 1 of the first embodiment, the guide device 100 of the third embodiment, the guide device 200 of the fifth embodiment, and the guide device 400 of the seventh embodiment.

Although the guide device of the present invention has been described based on the preferred embodiments, the present invention is not limited thereto. The configuration of each part can be replaced with any configuration having the same function. Further, any other means or components may be added to the present invention. Furthermore, the present invention may be a combination of any two or more configurations (features) of the above-described embodiments.

In addition, it has been described that the puncturing using the guide device of the present invention is performed by placing the guide device on the patient's body so that the distal end of the reference portion of the guide device matches the skin point B. However, the present invention is not limited thereto. As long as the puncture needle surely reaches the lumbar subarachnoid space, the position of the puncture point A, the position of the target point D and the position where the guide device is placed on the patient's body are not particularly limited. For example, the target point D may be positioned at the lumbar subarachnoid space between the fourth lumbar vertebra and the fifth lumbar vertebra.

**INDUSTRIAL APPLICABILITY**

The present invention provides a guide device for puncturing with a puncture needle from a puncture point A provided at a vicinity of a first point B on a waist of a patient to a target point D inside a patient's body, the target point D corresponding to a second point C different from the first point B on the waist. The guide device includes a guide part for guiding the puncturing with the puncture needle, and a support base for supporting the guide part and abutting on the waist. A portion of the support base is configured to define a first angle with respect to a straight line connecting the puncture point A and the second point C. Further, the guide part is provided at a second angle with respect to a line perpendicular to the support base. The first angle is defined by the puncture point A, the second point C and the first point B. The second angle is defined by the puncture point A, the target point D and the second point C. Accordingly, it is possible to provide a guide device capable of easily and accurately puncturing a target point with a puncture needle regardless of a patient and an operator. Thus, the present invention has industrial applicability.

**EXPLANATION OF REFERENCE NUMERALS**

1, 10, 100, 200, 300, 400, 500: guide device, 11: puncture needle, 2: guide part, 2a, 2b: sub-guide part, 21: main body, 211: reinforcing portion, 212: first support portion, 213: second support portion, 22: guide rail, 22a, 22b, 22c: sub-rail, 221: fitting portion, 23: grip portion, 25: plate member, 251: scale, 3: support base, 31: first blade portion, 311: reference portion, 312: notch, 313: puncture guide portion, 32: second blade portion, 321: reference portion, 322: notch, 323: puncture guide portion, 33: notch, 34: bottom portion, 35: marker, 8: angle changing device, 81: adjusting portion, 82: pivot portion, 83: fixing portion, 84: variation reference portion, 85: scale portion, 86: pressure portion, 861: pressure means, 862: fixing means, 87: rail portion, 9: fixing member, L1: extension line, L2: imaginary line

**Claims**

1. A guide device for puncturing with a puncture needle from a puncture point A provided at a vicinity of a first point B on a waist of a patient to a target point D inside a patient's body, the target point D corresponding to a second point C different from the first point B on the waist, the guide device comprising:

   a guide part for guiding the puncturing with the puncture needle; and
   a support base for supporting the guide part and abutting on the waist,
   wherein a portion of the support base is configured to define a first angle with respect to a straight line connecting the puncture point A and the second point C, and
   wherein the guide part is provided at a second angle with respect to a line perpendicular to the support base.

2. The guide device according to claim 1, wherein the first angle is defined by the puncture point A, the second point C and the first point B, and
   wherein the second angle is defined by the puncture point A, the target point D and the second point C.

3. The guide device according to claim 1 or 2, wherein the first angle is 20° to 40°.

4. The guide device according to any one of claims 1 to 3, wherein the second angle is 10° to 50°.

5. The guide device according to any one of claims 1 to 4, wherein the first angle and the second angle are determined by obtaining at least a CT image of the patient including the puncture point A, a CT image of the patient including the target point D, and CT images of the patient between the CT image of the patient including the puncture point A and the CT image of the patient including the target point D.

6. The guide device according to any one of claims 1 to 5, wherein the support base is located along a median line of the waist and has a reference portion having a predetermined length.

7. The guide device according to claim 6, wherein the support base further includes:

   a notch through which the puncture needle passes; and
   a puncture guide portion which is provided between the reference portion and the notch and is configured to guide the puncture point A through which the puncture needle is inserted into the patient's body.

8. The guide device according to claim 7, wherein the puncture guide portion is orthogonal to the reference portion and has a predetermined length.

9. The guide device according to any one of claims 1 to 8, further comprising a first angle changing device for changing the first angle and/or a second angle changing device for changing the second angle.

10. The guide device according to any one of claims 1 to 9, wherein the guide part has a plurality of guide parts, and wherein the second angle includes a plurality of different second angles, and the plurality of guide parts are provided to be supported by the support base with the different second angles respectively.

11. A method of manufacturing a guide device for puncturing with a puncture needle into a waist of a patient, the method comprising:

irradiating the waist with X-rays to obtain CT images of the waist;
setting a first point B on a median line of the patient, a puncture point A positioned at a vicinity of the first point B, a second point C different from the first point B on the median line and a target point D in the waist corresponding to the second point C to the CT images;
calculating a first angle $\angle BCA(\theta 1)$ and a second angle $\angle CDA(\theta 2)$;
preparing a support base designed to define the first angle and a guide part designed to define the second angle; and
connecting the guide part to the support base so as to set an angle with respect to a line perpendicular to the support base to the second angle.

12. The method of manufacturing the guide device according to claim 11, further comprising
changing the angle of the guide part connected to the support base in accordance with the second angle of the patient.

【FIG.1】

FIG.1

【FIG.2】

A    B    D

FIG.2

【FIG.3】

FIG.3

【FIG.4】

FIG.4

【FIG.5】

FIG.5

【FIG.6】

```
                    ┌─────────────────────────────┐  ⌐S1
                    │      PERFORM LUMBAR CT       │
                    └─────────────────────────────┘
                                  │
          ┌──────────────────────────────────────────┐  ⌐S2
          │          SET POINTS A TO D                │
          │            ON CT IMAGES                   │
          └──────────────────────────────────────────┘
                                  │
       ┌────────────────────────────────────────────────┐  ⌐S3
       │     CALCULATE FIRST ANGLE AND SECOND ANGLE      │
       └────────────────────────────────────────────────┘
                                  │
     ┌───────────────────────────────────────────────────────┐  ⌐S4
     │  SET FIRST ANGLE ON EXTENSION LINE OF REFERENCE PORTION │
     │  AND ADJUST SUPPORT BASE                                │
     └───────────────────────────────────────────────────────┘
                                  │
   ┌─────────────────────────────────────────────────────────────┐  ⌐S5
   │  SET ANGLE OF GUIDE RAIL WITH RESPECT TO LINE PERPENDICULAR TO│
   │  SUPPORT BASE TO SECOND ANGLE                                 │
   └─────────────────────────────────────────────────────────────┘
                                  │
        ┌──────────────────────────────────────┐  ⌐S6
        │      BRING GUIDE DEVICE INTO          │
        │     CONTACT WITH PATIENT'S BODY       │
        └──────────────────────────────────────┘
                                  │
     ┌────────────────────────────────────────────┐  ⌐S7
     │   ADVANCE PUNCTURE NEEDLE ALONG GUIDE RAIL  │
     └────────────────────────────────────────────┘
                                  │
          ┌───────────────────────────────┐  ⌐S8
          │     PUNCTURE PATIENT'S BODY    │
          │      WITH PUNCTURE NEEDLE      │
          └───────────────────────────────┘
```

# FIG.6

31

【FIG.7】

FIG.7

【FIG.8】

FIG.8

【FIG.9】

```
┌─────────────────────────────────────────────────┐
│ S11        PERFORM LUMBAR CT                      │
└─────────────────────────────────────────────────┘
                        │
┌─────────────────────────────────────────────────┐
│ S12     OBTAIN SLICE IMAGES OF LUMBAR             │
│         SPINAL CANAL                              │
└─────────────────────────────────────────────────┘
                        │
┌─────────────────────────────────────────────────┐
│ S13   IMAGE PROCESSING ON SLICE IMAGES           │
└─────────────────────────────────────────────────┘
                        │
┌─────────────────────────────────────────────────┐
│ S14 IDENTIFY PUNCTURE SLICE IMAGE BETWEEN SLICE   │
│ IMAGE INCLUDING FOURTH LUMBAR VERTEBRA AND SLICE  │
│ IMAGE INCLUDING FIFTH LUMBAR VERTEBRA             │
└─────────────────────────────────────────────────┘
                        │
┌─────────────────────────────────────────────────┐
│ S14A  IMAGE PROCESSING AND SHAPE ANALYSIS OF      │
│ PUNCTURE SLICE IMAGE                              │
└─────────────────────────────────────────────────┘
                        │
┌─────────────────────────────────────────────────┐
│ S15   SET PUNCTURE POINT A                        │
└─────────────────────────────────────────────────┘
                        │
┌─────────────────────────────────────────────────┐
│ S16 IDENTIFY TARGET SLICE IMAGE BETWEEN SLICE     │
│ IMAGE INCLUDING THIRD LUMBAR VERTEBRA AND SLICE   │
│ IMAGE INCLUDING FOURTH LUMBAR VERTEBRA            │
└─────────────────────────────────────────────────┘
                        │
┌─────────────────────────────────────────────────┐
│ S17   IMAGE PROCESSING AND SHAPE ANALYSIS OF      │
│ TARGET SLICE IMAGE                                │
└─────────────────────────────────────────────────┘
                        │
┌─────────────────────────────────────────────────┐
│ S18   SET TARGET POINT D                          │
└─────────────────────────────────────────────────┘
                        │
┌─────────────────────────────────────────────────┐
│ S19 IDENTIFY PASSING POINTS ON PLURALITY OF       │
│ SLICE IMAGES ON STRAIGHT LINE CONNECTING          │
│ PUNCTURE POINT A AND TARGET POINT D               │
└─────────────────────────────────────────────────┘
                        │
              ◇ S20 DOES EACH OF PASSING
     Yes ─────   POINTS COLLIDE WITH BONE? ◇
                        │ No
┌─────────────────────────────────────────────────┐
│ S21   CALCULATE ANGLES                            │
└─────────────────────────────────────────────────┘
                        │
┌─────────────────────────────────────────────────┐
│ S22 SPECIFY HORIZONTAL REACHABLE RANGE IN         │
│ PLURALITY OF SLICE IMAGES ON STRAIGHT LINE        │
│ CONNECTING PUNCTURE POINT A AND TARGET POINT D    │
└─────────────────────────────────────────────────┘
```

S23   SPECIFY ENTIRE REACHABLE RANGE OF PUNCTURE ROUTE

S22A CALCULATE AVERAGE OF ANGLES

S23A CALCULATE ANGLES OF PUNCTURE ROUTE AND ITS RANGE THAT REACHABLE TO TARGET AREA

S24   PREPARE GUIDE PART AND SUPPORT BASE

S25   CONNECT GUIDE PART TO SUPPORT BASE

FIG.9

【FIG.10】

(a)                (b)

# FIG.10

【FIG.11】

TARGET POINT D
(POINT IN SPINAL CANAL)

PUNCTURE POINT A

PUNCTURE NEEDLE

PASSING POINTS

# FIG.11

【FIG.12】

DETERMINE BONE SECTION
(WHITE SECTION) BASED ON
BRIGHTNESS

FIG.12

【FIG.13】

(a)                    (b)

FIG.13

【FIG.14】

FIG.14

【FIG.15】

FIG.15

【FIG.16】

(a) POSITIONAL RELATIONSHIP BETWEEN POINTS A, B, C, D AND Θ1, Θ2

(b) POSITIONAL RELATIONSHIP WITH REGARD TO Θ1

(c) POSITIONAL RELATIONSHIP WITH REGARD TO Θ2

(d) POSITIONAL RELATIONSHIP WITH REGARD TO Θ1

FIG.16

【FIG.17】

S13

S141 IDENTIFY PUNCTURE SLICE FROM CANDIDATE IMAGES

S141A IMAGE PROCESSING AND SHAPE ANALYSIS OF PUNCTURE SLICE IMAGES

S151A SET PUNCTURE POINT A ON EACH SLICE

S161 IDENTIFY TARGET SLICE IMAGES

S171 IMAGE PROCESSING AND SHAPE ANALYSIS OF TARGET SLICE IMAGES

S181 SET PLURALITY OF TARGET POINTS D

S182 CREATE PLURALITY OF PUNCTURE ROUTES CONNECTING PLURALITY OF PUNCTURE POINT CANDIDATES AND PLURALITY OF TARGET POINT CANDIDATES

S191 IDENTIFY PASSING POINTS ON PLURALITY OF SLICE IMAGES ON EACH ROUTE IN PLURALITY OF ROUTE CANDIDATES

S201 DO PASSING POINTS OF EACH OF ROUTE CANDIDATES COLLIDE WITH BONE?

Yes → S202 DETERMINE THAT IT IS NOT CANDIDATE

No

S221 FOR ALL ROUTE CANDIDATES WHICH DO NOT COLLIDE WITH BONE, SPECIFY HORIZONTAL REACHABLE RANGE IN TARGET SLICE IMAGE

S231 SPECIFY REACHABLE RANGE OF PUNCTURE NEEDLE

S231A CALCULATE AREA OF REACHABLE RANGE (SUBSTANTIALLY ELLIPSE SHAPE)

S231B SPECIFY ROUTE HAVING LARGEST AREA OF REACHABLE RANGE AND CALCULATE ANGLES

S24

FIG.17

【FIG.18】

FIG.18

【FIG.19】

FIG.19

【FIG.20】

FIG.20

【FIG.21】

FIG.21

【FIG.22】

FIG.22

【FIG.23】

FIG.23

【FIG.24】

FIG.24

【FIG.25】

FIG.25

【FIG.26】

FIG.26

## INTERNATIONAL SEARCH REPORT

| International application No. |
|---|
| **PCT/JP2021/028462** |

### A. CLASSIFICATION OF SUBJECT MATTER

*A61B 17/34*(2006.01)i; *A61M 5/42*(2006.01)i
FI:   A61B17/34; A61M5/42 520

According to International Patent Classification (IPC) or to both national classification and IPC

### B. FIELDS SEARCHED

Minimum documentation searched (classification system followed by classification symbols)

A61B17/34; A61M5/42

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| A | US 2019/0183523 A1 (EDWARD VIA COLLEGE OF OSTEOPATHIC MEDICINE) 20 June 2019 (2019-06-20)<br>fig. 1-7 | 1-12 |
| A | US 2016/0022308 A1 (THE UNIVERSITY OF BRITISH COLUMBIA) 28 January 2016 (2016-01-28)<br>fig. 1-8A | 1-12 |
| A | CN 101926679 A (GOU, Dongwei) 29 December 2010 (2010-12-29)<br>fig. 1-10 | 1-12 |

☐ Further documents are listed in the continuation of Box C.     ☑ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **01 October 2021** | **12 October 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT
**Information on patent family members**

International application No.

**PCT/JP2021/028462**

| Patent document cited in search report | | | Publication date (day/month/year) | Patent family member(s) | | | Publication date (day/month/year) |
|---|---|---|---|---|---|---|---|
| US | 2019/0183523 | A1 | 20 June 2019 | US fig. 1-7 | 2016/0324539 | A1 | |
| US | 2016/0022308 | A1 | 28 January 2016 | WO fig. 1-8A | 2014/138918 | A1 | |
| CN | 101926679 | A | 29 December 2010 | (Family: none) | | | |

Form PCT/ISA/210 (patent family annex) (January 2015)

**REFERENCES CITED IN THE DESCRIPTION**

**Patent documents cited in the description**

- JP H7250902 A

- JP 2019213662 A